# EUROPÄISCHE PATENTSCHRIFT

(11) **EP 0 657 173 B1**
(45) Veröffentlichungstag und Bekanntmachung des Hinweises auf die Patenterteilung: **12.05.1999**
(21) Anmeldenummer: 94118681.9
(22) Anmeldetag: 28.11.1994
(51) Int. Cl.: A61K 38/15

(54) **Endoparasitizide Mittel auf Basis von offenkettigen Hexadepsipeptiden**
Endoparasiticide drugs based upon open chain hexadepsipeptides
Remèdes endoparasiticides à base de hexadepsipeptides chaînes ouvertes

(30) Priorität: 09.12.1993 DE 4341991
(43) Veröffentlichungstag der Anmeldung: 14.06.1995
(73) Patentinhaber: BAYER AG, 51368 Leverkusen (DE)
(72) Erfinder: Jeschke, Peter, Dr., D-51373 Leverkusen (DE); Scherkenbeck, Jürgen, Dr., D-42929 Wermelskirchen (DE); Plant, Andrew, Dr., D-51519 Odenthal (DE); Harder, Achim, Dr., D-51109 Köln (DE); Mencke, Norbert, Dr., D-51381 Leverkusen (DE)

(56) Entgegenhaltungen:
- WO-A-93/25543
- DE-A- 4 317 458

## Beschreibung

Die vorliegende Erfindung betrifft die Verwendung von offenkettigen Hexadepsipeptiden zur Herstellung eines Arzneimittels zur Bekämpfung von Endoparasiten.

Bestimmte offenkettige Hexadepsipeptide sind als Ausgangsstoffe für endoparasitizid wirksame cyclische Depsipeptide mit 18 Ringatomen (Enniatine) Gegenstand einer vorgängigen, jedoch nicht vorveröffentlichten Patentanmeldung (Deutsche Patentanmeldung DE-OS 4 317 458.2).

Über eine Verwendung dieser Verbindungen gegen Endoparasiten ist jedoch bisher nichts bekannt geworden.

Es wurde nun gefunden, daß die offenkettigen Hexadepsipeptide der allgemeinen Formel (I) 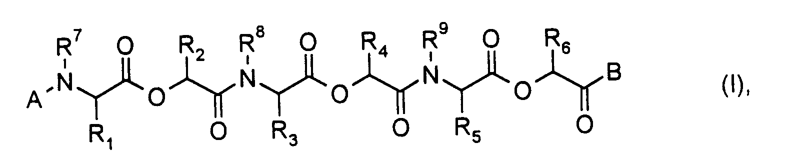 in welcher
- A: für Wasserstoff, Alkyl, Aralkyl oder einem Arylrest, insbesondere für einen Rest der Formel -CO-R¹⁰ steht, worin
- R¹⁰: für geradkettiges, verzweigtes Alkyl, Aralkyl, Alkoxy oder Arylalkoxy mit bis zu 6 Kohlenstoffatomen im Alkylteil steht,
- R¹, R³ und R⁵: unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein k ann, Alkoxycarbonylaminoalkyl, 9-Fluorenylmethoxycarbonyl(Fmoc)aminoalkyl, Alkenyl, Cycloalkyl, Cycloalkylalkyl, gegebenenfalls substituiertes Aryl, Hetarylmethyl sowie Arylalkyl, wobei als Substituenten genannt seien Halogen, Hydroxy, Alkyl, Alkoxy, Nitro oder ein Rest -NR¹¹R¹², wobei R¹¹ und R¹² unabhängig voneinander für Wasserstoff oder Alkyl stehen oder R¹¹R¹² gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch C₁-C₄-Alkyl substituiert ist,
- R², R⁴ und R⁶: unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Alkylthioalkyl, Alkylsulfinylalkyl, Alkysulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl, Hetarylmethyl sowie gegebenenfalls substituiertes Arylalkyl, wobei als Substituenten genannt seien Halogen, Hydroxy, Alkyl, Alkoxy, Nitro oder ein Rest -NR¹¹R¹², wobei R¹¹ und R¹² unabhängig voneinander für Wasserstoff oder Alkyl stehen oder R¹¹R¹² gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch C₁-C₄-Alkyl substituiert ist,
- R⁷, R⁸ und R⁹: unabhängig voneinander für Wasserstoff, C₁₋₈-Alkyl, C₃₋₆-Cycloalkyl oder für Aralkyl stehen,
- B: für Hydroxy oder Alkoxy mit bis zu 4 Kohlenstoffatomen steht,
sowie deren optische Isomere und Racemate,
zur Herstellung eines Arzneimittels zur Bekämpfung von Endoparasiten in der Medizin und Tiermedizin Verwendung finden können.

Die erfindungsgemäß zu verwendenden offenkettigen Hexadepsipeptide sind durch die Formel (I) allgemein definiert. Vorzugsweise werden erfindungsgemäß diejenigen Verbindungen der Formel (I) verwendet
in welcher
- A: für Wasserstoff, C₁₋₄-Alkyl, Benzyl oder für eine Gruppe der Formel -CO-R¹⁰ steht,
worin
- R¹⁰: für geradkettiges oder verzweigtes C₁₋₄-Alkyl, C₂₋₄-Alkenyl, C₁₋₄-Alkoxy oder Phenylalkoxy mit bis zu 6 Kohlenstoffatomen im Alkylteil, insbesondere für tert.-Butoxy, Benzyloxy, Ethoxy, Allyloxy, Fluorenyl-9-methoxy oder Methoxy steht,
- R¹, R³ und R⁵: unabhängig voneinander für Wasserstoff geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Octyl, Isooctyl, sec.-Octyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl(Fmoc)aminopropyl, 9-Fluorenylmethoxycarbonyl(Fmoc)aminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Aryl, insbesondere Phenyl, Hetarylmethyl, insbesondere Benzo[b]thien-1-yl-methyl, Benzo[b]thien-3-yl-methyl, Naphth-1-yl-methyl, Naphth-2-yl-methyl, Pyrid-2-yl-methyl, Pyrid-3-yl-methyl, Fur-2-yl-methyl, Fur-3-yl-methyl, Thien-2-yl-methyl, Thien-3-yl-methyl, Indol-3-yl-methyl, N-Methyl-indol-3-yl-methyl, Imidazol-4-yl-methyl, N-Methyl-imidazol-4-yl-methyl, Phenyl-C₁-C₄-alkyl, insbesondere Phenylmethyl, die gegebenenfalls durch Reste aus der Reihe Halogen, insbesondere Fluor, Chlor, Brom oder Iod, Hydroxy, C₁-C₄-Alkyl, insbesondere Methyl oder tert.-Butyl, C₁-C₄-Alkoxy, insbesondere Methoxy, Ethoxy, Methylendioxy oder Ethylendioxy, Nitro oder C₁-C₄-Alkylamino, insbesondere Methylamino, C₁-C₄-Dialkylamino, insbesondere Dimethylamino, C₃-C₆-Cycloalkylamino, insbesondere Piperidino oder Pyrrolidino, C₃-C₆-Cycloalkoxyamino, insbesondere Morpholino, C₃-C₆-Cycloalkylthioxyamino, insbesondere Thiomorpholino, substituiert sein können,
- R², R⁴ und R⁶: unabhängig voneinander für Wasserstoff geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Octyl, Isooctyl, sec.-Octyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl(Fmoc)aminopropyl, 9-Fluorenylmethoxycarbonyl(Fmoc)aminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Aryl, insbesondere Phenyl, Hetarylmethyl, insbesondere Benzo[b]thien-1-yl-methyl, Benzo[b]thien-3-yl-methyl, Naphth-1-yl-methyl, Naphth-2-yl-methyl, Pyrid-2-yl-methyl, Pyrid-3-yl-methyl, Fur-2-yl-methyl, Fur-3-yl-methyl, Thien-2-yl-methyl, Thien-3-yl-methyl, Indol-3-yl-methyl, N-Methyl-indol-3-yl-methyl, Imidazol-4-yl-methyl, N-Methyl-imidazol-4-yl-methyl, Phenyl-C₁-C₄-alkyl, insbesondere Phenylmethyl, das gegebenenfalls durch Reste aus der Reihe Halogen, insbesondere Fluor, Chlor, Brom oder Iod, Hydroxy, C₁-C₄-Alkyl, insbesondere Methyl oder tert.-Butyl, C₁-C₄-Alkoxy, insbesondere Methoxy, Ethoxy, Methylendioxy oder Ethylendioxy, Nitro oder C₁-C₄-Alkylamino, insbesondere Methylamino, C₁-C₄-Dialkylamino, insbesondere Dimethylamino, C₃-C₆-Cycloalkylamino, insbesondere Piperidino oder Pyrrolidino, C₃-C₆-Cycloalkoxyamino, insbesondere Morpholino, C₃-C₆-Cycloalkylthioxyamino, insbesondere Thiomorpholino, substituiert sein kann,
- R⁷, R⁸ und R⁹: unabhängig voneinander für Wasserstoff, geradkettiges oder verzweigtes C₁-C₄-Alkyl, insbesondere Methyl, Ethyl, Isopropyl, t-Butyl, s-Butyl, Benzyl, Cyclopropyl oder Cyclohexyl stehen,
- B: für Hydroxy oder tert.-Butyloxy steht,
sowie deren optische Isomere und Racemate.

Besonders bevorzugt werden erfindungsgemäß diejenigen Verbindungen der Formel (I) verwendet
in welcher
- A: für Wasserstoff oder Benzyl steht,
oder für eine Gruppe der Formel -CO-R¹⁰ steht,
worin
- R¹⁰: für geradkettiges oder verzweigtes Alkoxy oder Arylalkoxy mit bis zu 6 Kohlenstoffatomen im Alkylteil, insbesondere für tert.-Butoxy, Benzyloxy steht,
- R¹, R³ und R⁵: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, Hydroxy-C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₂-C₈-Alkenyl, insbesondere Vinyl, Allyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Aryl, insbesondere Phenyl, Hetarylmethyl, insbesondere Naphth-1-yl-methyl, Pyrid-2-yl-methyl, Pyrid-3-yl-methyl, Thien-2-yl-methyl, Thien-3-yl-methyl, Indol-3-yl-methyl, N-Methyl-indol-3-yl-methyl, Imidazol-4-yl-methyl, N-Methyl-imidazol-4-yl-methyl, Phenyl-C₁-C₄-alkyl, insbesondere Phenylmethyl, das gegebenenfalls durch einen oder mehrere gleiche oder verschiedene der oben angegebenen Reste substituiert sein kann,
- R², R⁴ und R⁶: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, Hydroxy-C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₂-C₈-Alkenyl, insbesondere Vinyl, Allyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Aryl, insbesondere Phenyl, Hetarylmethyl, insbesondere Benzo[b]thien-2-yl-methyl, Naphth-1-yl-methyl, Naphth-2-yl-methyl, Pyrid-2-yl-methyl, Pyrid-3-yl-methyl, Fur-2-yl-methyl, Fur-3-yl-methyl, Thien-2-yl-methyl, Thien-3-yl-methyl, Phenyl-C₁-C₄-alkyl, insbesondere Phenylmethyl, das gegebenenfalls durch einen oder mehrere gleiche oder verschiedene der oben angegebenen Reste substituiert sein kann,
- R⁷, R⁸ und R⁹: unabhängig voneinander für Wasserstoff, Methyl, Benzyl oder Cyclopropyl stehen,
- B: für Hydroxy oder tert.-Butyloxy steht,
sowie deren optische Isomere und Racemate.

Ganz besonders bevorzugt werden erfindungsgemäß diejenigen Verbindungen der Formel (I) verwendet,
in welcher
- A: für Wasserstoff, Benzyl, tert.-Butyloxycarbonyl (Boc) oder Benzyloxycarbonyl (Z) steht,
- R¹, R³ und R⁵: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, C₂-C₈-Alkenyl, insbesondere Allyl, C₃-C₇-Cycloalkyl-C₁-C₄-alkyl, insbesondere Cyclohexylmethyl, Phenyl-C₁-C₄-alkyl, insbesondere Phenylmethyl,
- R², R⁴ und R⁶: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, C₂-C₈-Alkenyl, insbesondere Vinyl, Allyl, C₃-C₇-Cycloalkyl-C₁-C₄-alkyl, insbesondere Cyclohexylmethyl, Phenyl-C₁-C₄-alkyl, insbesondere Phenylmethyl, das gegebenenfalls durch einen oder mehrere gleiche oder verschiedene der oben angegebenen Reste substituiert sein kann,
- R⁷, R⁸ und R⁹: für Methyl stehen,
- B: für Hydroxy oder tert.-Butyloxy steht,
sowie deren optische Isomere und Racemate.

Die Herstellung der erfindungsgemäß zu verwendenden offenkettigen Hexadepsipeptide der allgemeinen Formel (I) ist Gegenstand einer vorgängigen, jedoch nicht vorveröffentlichten Patentanmeldung der Anmelderin (Deutsche Patentanmeldung P 4 317 458-2).

Man erhält die Verbindungen der Formel (I) 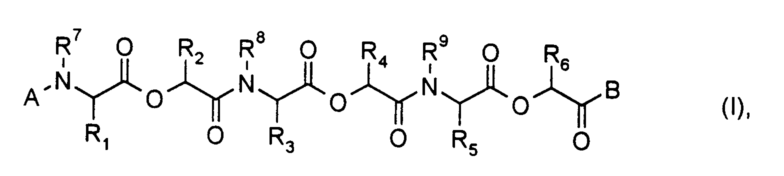 in welcher
A, R¹, R², R³, R⁴, R⁵, R⁶, R⁷, R⁸ und R⁹ und B die weiter oben angegebene Bedeutung besitzen, wenn man beispielsweise

### a) Tetradepsipeptide der allgemeinen Formel (IIb)

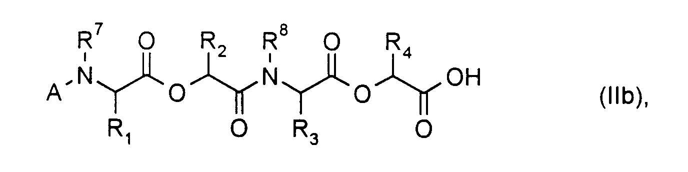

in welcher
A, R¹, R², R³, R⁴, R⁷ und R⁸ die oben angegebene Bedeutung haben,
mit Didepsipeptiden der allgemeinen Formel (VIc) 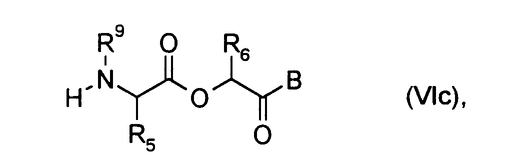 in welcher
B, R⁵, R⁶ und R⁹ die weiter oben angegebene Bedeutung besitzen,
in Gegenwart geeigneter Kupplungsreagenzien, in Gegenwart eines basischen Reaktionshilfsmittels und in Gegenwart eines Verdünnungsmittels umsetzt oder

### b) Tetradepsipeptide der allgemeinen Formel (IIIc)

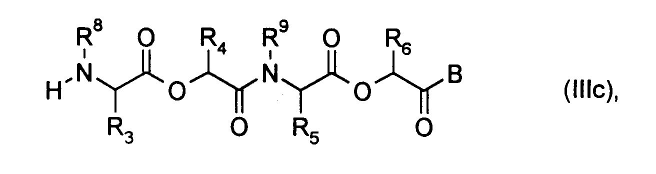

in welcher
B, R³, R⁴, R⁵, R⁶, R⁸ und R⁹ die weiter oben angegebene Bedeutung besitzen,
mit Didepsipeptiden der allgemeinen Formel (IVb) 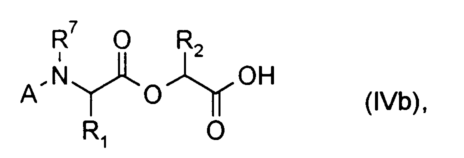 in welcher
A, R¹, R² und R⁷ die weiter oben angegebene Bedeutung besitzen,
in Gegenwart geeigneter Kupplungsreagenzien, in Gegenwart eines basischen Reaktionshilfsmittels und in Gegenwart eines Verdünnungsmittels umsetzt.

Die Verbindungen der allgemeinen Formel (I) können in optische aktiven, stereoisomeren Formen oder als racemische Gemische vorliegen und verwendet werden. Vorzugsweise werden jedoch die optisch aktiven, stereoisomeren Formen in Verbindung der allgemeinen Formel (I) erfindungsgemäß verwendet.

Im einzelnen seien folgende Verbindungen der allgemeinen Formel (I) genannt, in welcher die Reste R⁷ bis R⁹ für Methyl stehen und die Reste A, R¹ bis R⁶ und B die folgende Bedeutung haben:

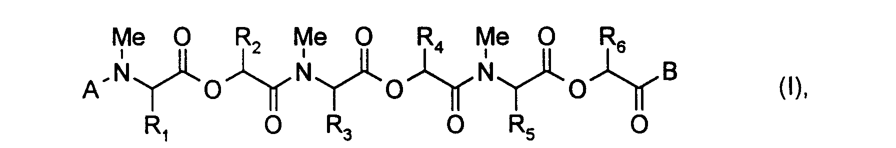

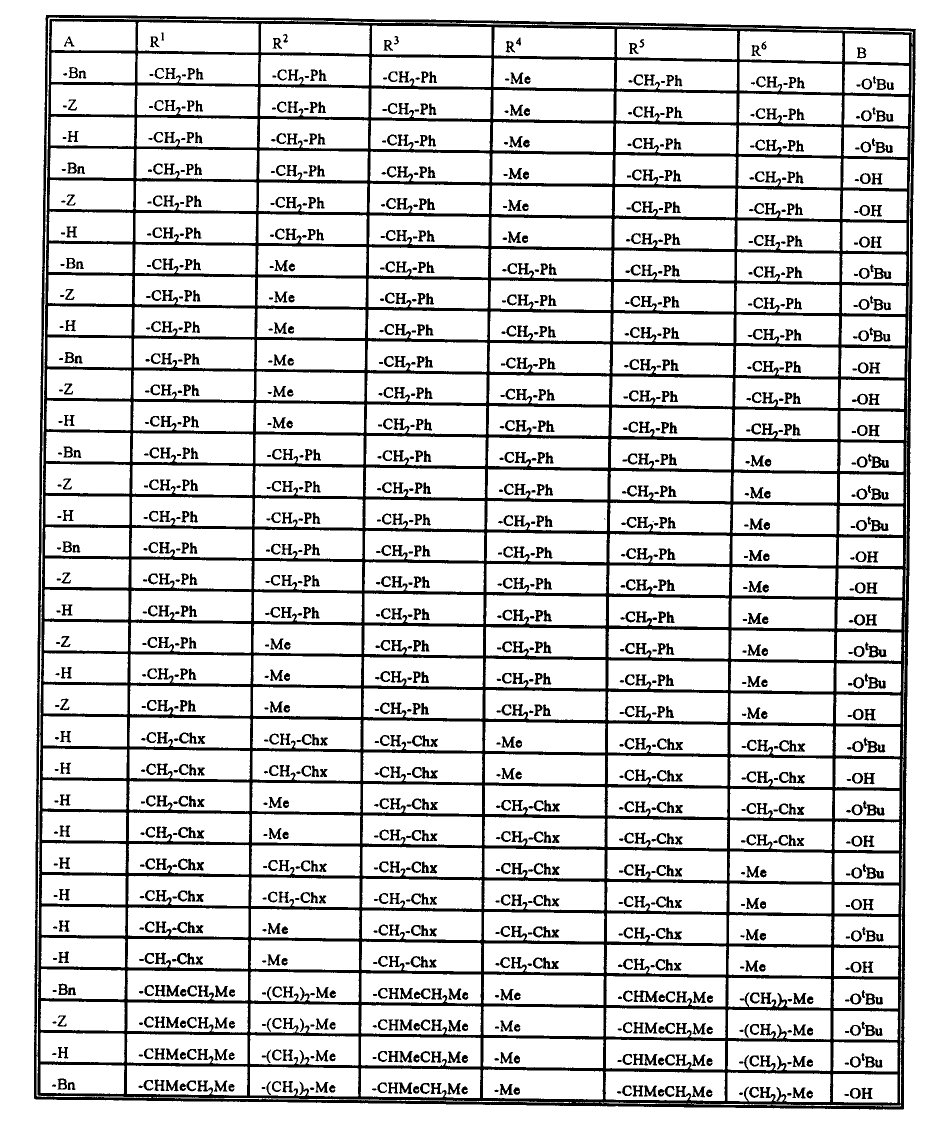

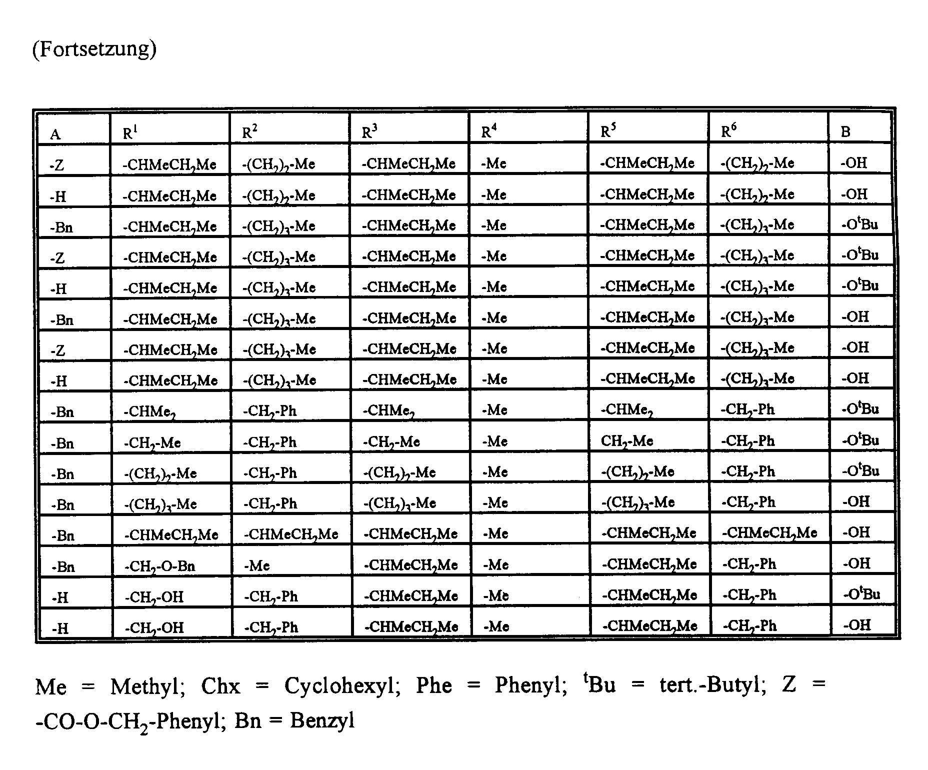

Die erfindungsgemäß verwendbaren Depsipeptide und ihre Ausgangsverbindungen können nach klassischen Verfahren hergestellt werden, beispielweise demjenigen, wie es von H.-G. Lerchen und H. Kunz (Tetrahedron Lett. 26 (43) (1985) S. 5257-5260; 28 (17) (1987) S. 1873-1876) unter Ausnutzung der Veresterungsmethode nach B.F. Gisin (Helv. Chim. Acta 56 (1973) S. 1476) beschrieben ist.

Die als Ausgangsmaterialien verwendeten N-Methyl-aminosäuren und 2-Halogencarbonsäurederivate sind teilweise bekannt (vgl. z.B. N-Methyl-aminosäuren: R. Bowmann et al. J. Chem. Soc. (1950) S. 1346; J.R. McDermott et al. Can. J. Chem. 51 (1973) S. 1915; H. Wurzinger et al., Kontakte (Merck. Darmstadt) 3 (1987) S. 8; 2-Halogencarbonsäurederivate: S.M. Birnbaum et al. J. Amer. Chem. Soc. 76 (1954) S. 6054, C.S. Rondestvedt, Jr. et al. Org. Reactions 11 (1960) S. 189 [Review]) bzw. können nach den dort beschriebenen Verfahren erhalten werden.

Für die Kupplungsreaktion zur Darstellung der als Ausgangsverbindungen eingesetzten Depsipeptide (II), (III), (IV), (V) und (VI) finden alle Kupplungsreagenzien, die zur Herstellung einer Amidbindung geeignet sind (vgl. z.B. Houben-Weyl, Methoden der organischen Chemie, Band 15/2; Bodanszky et al., Peptide Synthesis 2nd ed. (Wiley & Sons, New York 1976) oder Gross, Meienhofer, The Peptides: Analysis synthesis, biology (Academic Press, New York 1979), Verwendung.

Die der Erfindung entsprechenden offenkettigen Hexadepsipeptide der Formel (I), in welcher die Reste R⁷ bis R⁹ für Methyl stehen, können somit nach der folgenden Reaktionssequenz erhalten werden:

### a) Synthese der Didepsipeptide der Formeln (IV) bis (VI):

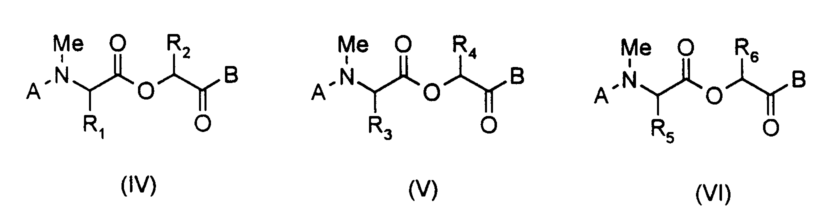

worin A eine N-terminale Schutzgruppe, wie z.B. die Benzyl oder Benzyloxycarbonylgruppe und B eine C-terminale Schutzgruppe, wie z.B. die tert.-Butoxygruppe, darstellt.

Dies entspricht beispielsweise für Formel (VIa) dem folgenden Reaktionsschema:

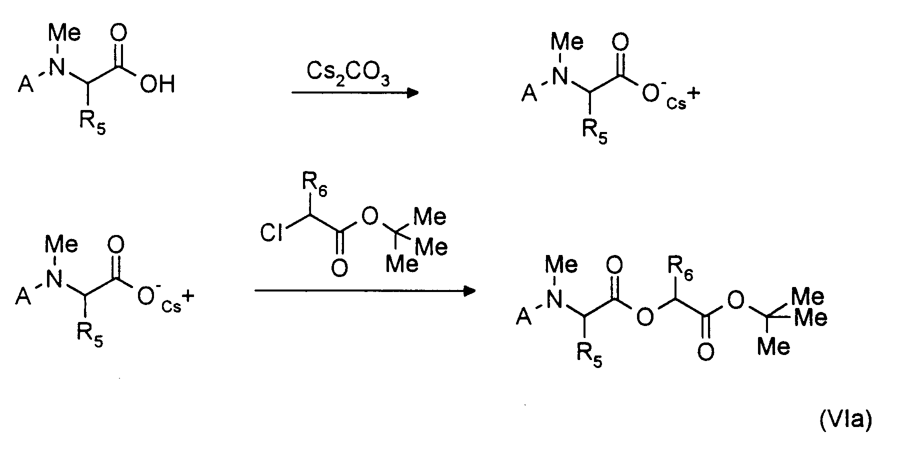

Die Herstellung der enantionmerenreinen Verbindungen der Formeln (IV), (V) und (VI) kann gegebenenfalls auch über die Trennung der Diastereomere nach üblichen Methoden wie beispielsweise Kristallisation, durch Säulenchromatographie oder durch Craig-Verteilung erfolgen. Welches das optimale Verfahren ist, muß von Fall zu Fall entschieden werden, manchmal ist es auch zweckmäßig, Kombinationen der einzelnen Verfahren zu benutzen.

Am Ende dieser Stufe kann entweder eine Entfernung der N-terminalen Schutzgruppe aus den Derivaten der Formel (VIa) in an sich bekannter Weise durchgeführt werden, beispielsweise durch katalytische Hydrierung, zur Herstellung der Derivate der Formel (VIc) oder es kann in an sich bekannter Weise eine Abspaltung der C-terminalen Schutzgruppe aus den Derivaten der Formel (IV) und (V), vorzugsweise durch Acidolyse, zur Darstellung der Derivate (IVb) und (Vb) erfolgen:

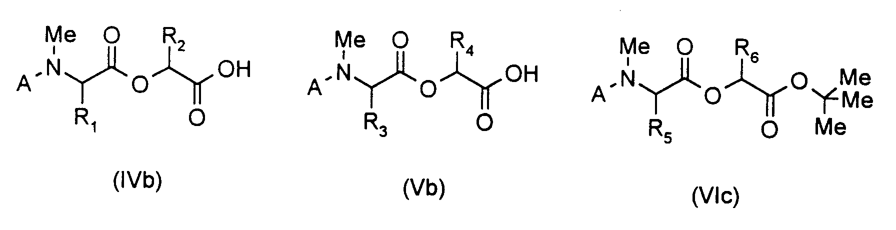

### b) Synthese der Tetradepsipeptide der Formel (II) und (III)

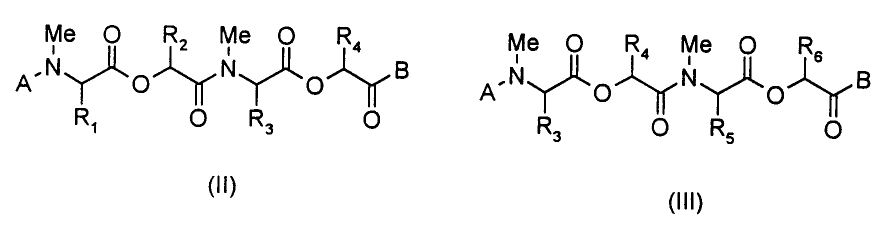

dies entspricht beispielsweise für Formel (IIIa) dem nachfolgenden Reaktionsschema:

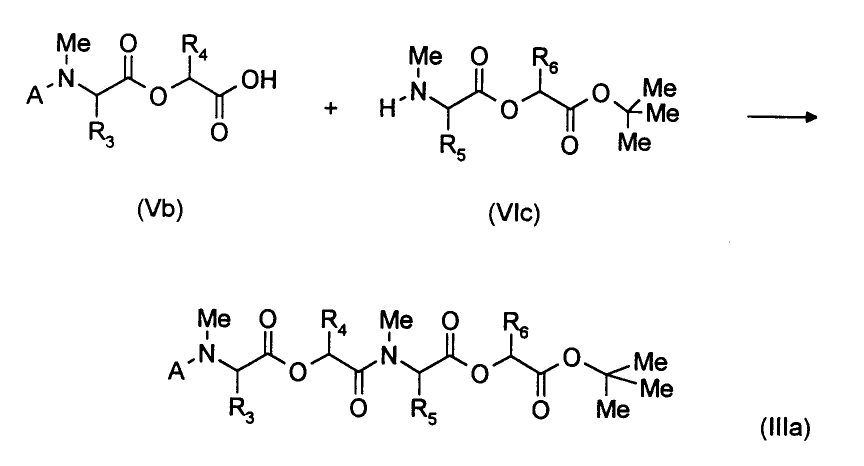

Man kann anschließend eine Entfernung der N-terminalen Schutzgruppe aus den Derivaten der Formel (IIIa) durchführen, beispielsweise durch katalytische Hydrierung wie oben angegeben, zur Herstellung der Derivate der Formel (IIIc)

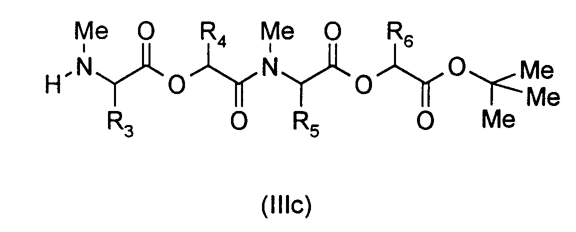

### c) Synthese der offenkettigen Hexadepsipeptide der Formel (I)

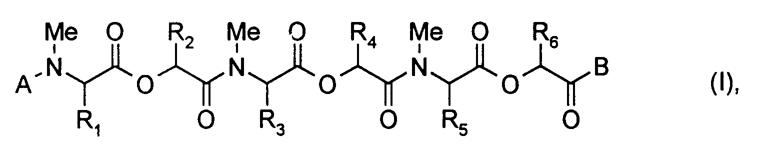

nach der folgenden Reaktionsgleichung:

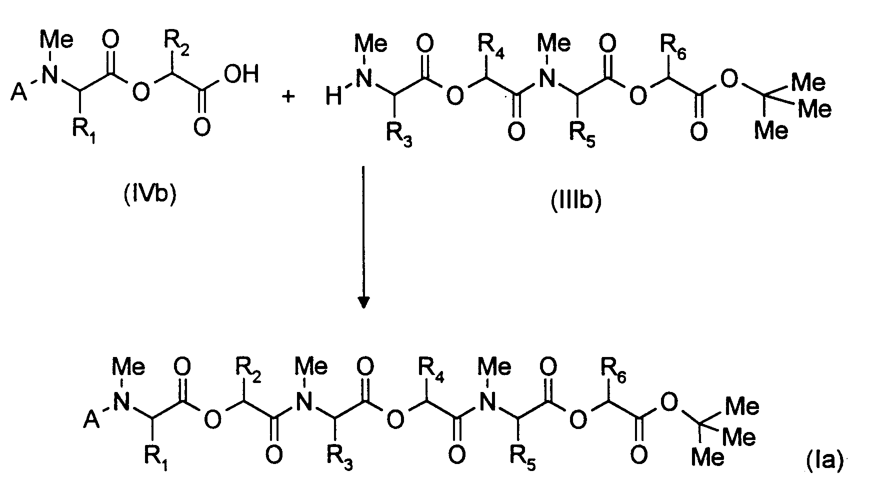

Man kann anschließend eine Entfernung der C-terminalen Schutzgruppe aus den Derivaten der Formel (Ia) in an sich bekannter Weise, beispielsweise durch Acidolyse, zur Herstellung der Derivate 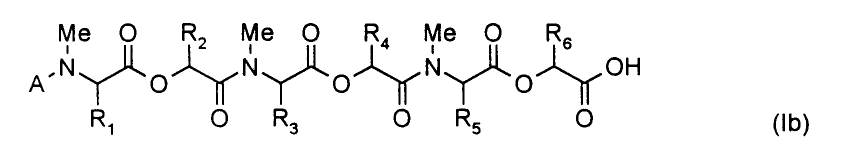 durchführen oder man deblockiert die Derivate der Formel (Ia) N-terminal in an sich bekannter Weise, beispielsweise durch katalytische Hydrierung wie oben angegeben, zur Herstellung der Derivate der Formel (Ic)

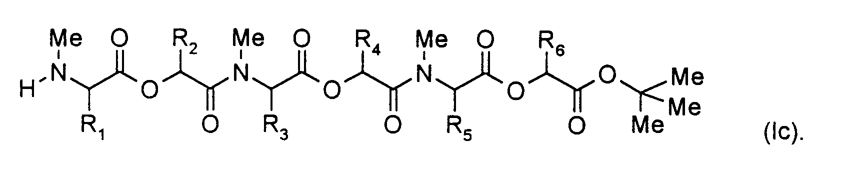

Am Ende dieser Stufen kann eine Entfernung der N-terminalen bzw. C-terminalen Schutzgruppe aus den Derivaten der Formel (Ib) bzw. (Ic) in an sich bekannter Weise durchgeführt werden, beispielsweise durch katalytische Hydrierung oder durch Acidolyse wie oben angegeben, zur Herstellung der Derivate der Formel (IId)

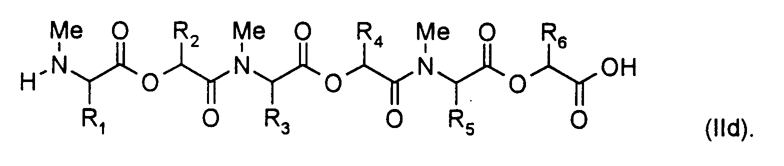

Die anfallenden Produkte lassen sich in üblicher Weise durch Umkristallisieren oder Säulenchromatographie reinigen (vgl. auch die Herstellungsbeispiele).

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: PHysiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl / Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleyl-alkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethynolaminsalz;
Als weitere Hilfsstoffe seien genannt: Viskositätserhöhende und die Emulsion stabilisierende Stoffe wie Carboxymethylcellulose, Methylcellulose und andere Cellulose-und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside ganannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

### Haemonchus contortus / Schaf

Experimentell mit Haemonchus contortus infizierte Schäft wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 3 | 10 |
| 6 | 10 |
| 14 | 10 |
| 43 | 10 |
| 52 | 10 |
| 63 | 10 |
| 65 | 10 |
| 67 | 10 |

### Herstellungsbeispiele

### Beispiel 1

### N-Benzyloxycarbonyl-N-methyl-L-isoleucyl-D-lactyl-N-methyl-L-isoleucyl-D-lactyl-N-methyl-L-isoleucyl-D-milchsäure-tert.-butylester

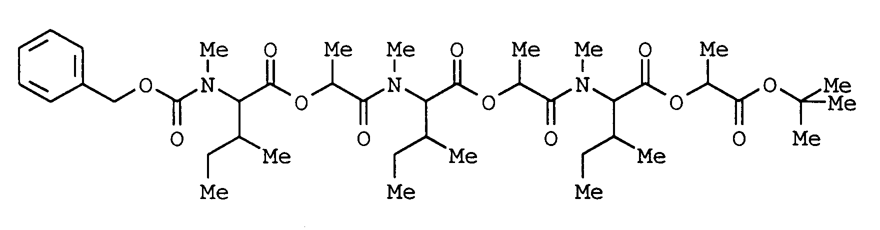

Zu einer Lösung von 5,8 g (16,5 mMol) Z-L-MeIle-D-Lac-OH und 7,8 g (16,5 mMol) H-(-L-MeIle-D-Lac-)₂-O-^{t}Bu in 150 ml Methylenchlorid werden bei 0 °C 4,7 g (36,3 mMol) N,N-Diisopropylethylamin ("Hünig's Base") sowie 4,6 g (18,1 mMol) Bis(2-oxo-3-oxazolidinyl)-phosphoniumsäurechlorid (BOP-Cl) zugegeben und 4 Stunden gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) mit dem Fließmittel Toluol : Essigsäureethylester (5:1) chromatographiert. Man erhält 10,3 g (77,4 % der Theorie) N-Benzyloxycarbonyl-N-methyl-L-isoleucyl-D-lactyl-N-methyl-L-isoleucyl-D-lactyl-N-methyl-L-isoleucyl-D-milchsäure-tert.-butylester.
- FAB-MS m/z (%):: 805 (M⁺,3); 749 (M⁺- H₂C=CMe₂,10); 732 (9); 793 (10); 91 (100)

### Beispiel 2

### N-Benzyl-N-methyl-L-isoleucyl-D-lactyl-N-methyl-L-isoleucyl-D-lactyl-N-methyl-L-phenylalanyl-D-milchsäure-tert.-butylester

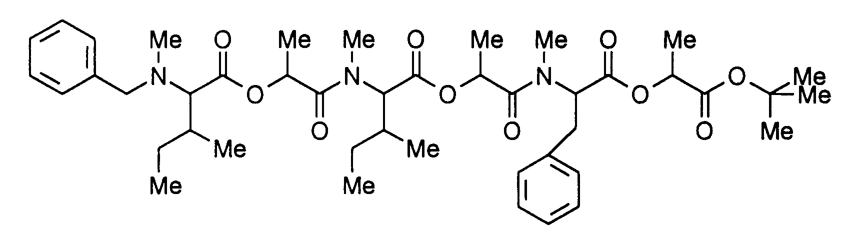

Die Kupplungsreaktion erfolgt analog der Reaktionsvorschrift des Beispiels 1 unter Verwendung von:
- 7,4 g (14,6 mMol): N-Benzyl-N-methyl-L-isoleucyl-D-lactyl-N-methyl-L-isoleucyl-D-milchsäure,
- 4,5 g (14,6 mMol): N-Methy-L-phenylalanyl-D-milchsäure-tert.-butylester,
- 150 ml: Methylenchlorid,
- 4,1 g (32,2 mMol): N,N-Diisopropylethylamin ("Hünig's Base"),
- 4,1 g (16,1 mMol): Bis(2-oxo-3-oxazolidinyl)-phosphoniumsäurechlorid (BOP-Cl).

Das zurückbleibende Rohprodukt wird zunächst über eine Kieselgelsäure (Kieselgel 60 - Merck, Korngröße: 0,04 bis 0,063 mm) mit dem Fließmittel Toluol:Essigsäureethylester (3:1) sowie Methylenchlorid:Methanol (8:1) und anschließend mit dem Fließmittel Toluol:Essigsäureethylester (10:1) chromatographiert.

Man erhält 5,2 g (44,6 % der Theorie) N-Benzyl-N-methyl-L-isoleucyl-D-lactyl-N-methyl-L-isoleucyl-D-lacyl-N-methyl-L-phenylalanyl-D-milchsäure-tert.-butylester.
- EI-MS m/z (%):: 794 (M⁺,1); 297 (8); 190 (100)

### Beispiel (II - 2)

### N-Benzyloxycarbonyl-N-methyl-L-isoleucyl-D-lactyl-N-methyl-L-isoleucyl-D-lactyl-N-methyl-L-isoleucyl-D-milchsäure

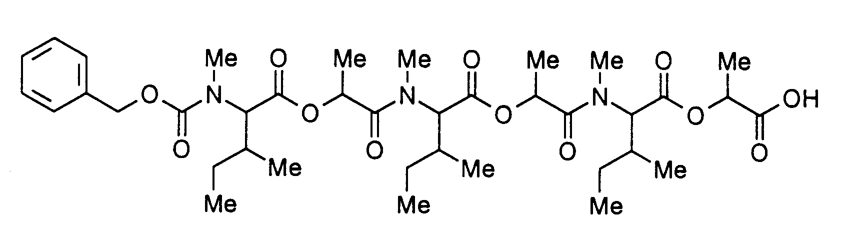

In eine auf 0 °C gekühlte Lösung von 9,2 g (11,2 mMol) Z-(-L-MeIle-D-Lac-)₃-O-^{t}Bu in 150 ml absolutem Methylenchlorid wird 20 Minuten trockenes Chlorwasserstoffgas eingeleitet. Anschließend rührt man ca. 16 Stunden bei Raumtemperatur und engt den gesamten Reaktionsansatz im Vakuum ein. Man erhält 7,1 g (82,9 % der Theorie) N-Benzyloxycarbonyl-N-methyl-L-isoleucyl-D-lactyl-N-methyl-L-isoleucyl-D-lactyl-N-methyl-L-isoleucyl-D-milchsäure.
- MS m/z (%):: 749 (M⁺,10); 721 (1); 693 (2); 533 (0.5); 91 (100)

### Beispiel 4

### N-Methyl-L-leucyl-D-phenyllactyl-N-methyl-L-leucyl-D-phenyllactyl-N-methyl-L-leucyl-D-milchsäure-tert.-butylester

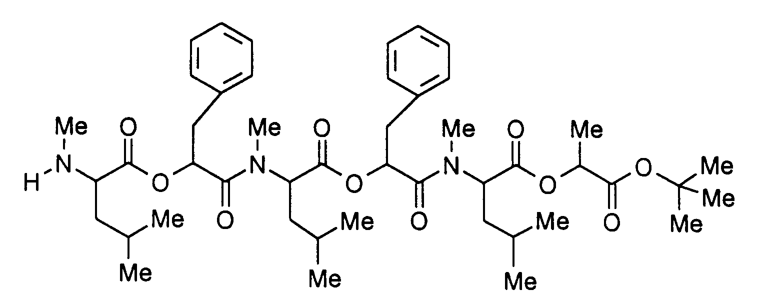

5,5 g (6,02 mMol) Bn-L-MeLeu-D-PheLac-L-MeLeu-D-PheLac-MeLeu-D-Lac-O^{t}Bu werden in 120 ml Ethanol in Gegenwart von 0,6 g Pd(OH)₂/Kohle [20 % Pd-Gehalt] bis zur Beendigung der Wasserstoffaufnahme hydriert (ca. 4 Stunden). Nach Abfiltrieren des Katalysators wird die gesamte Reaktionslösung im Vakuum eingeengt. Man erhält 4,8 g (97 % der Theorie) N-Methyl-L-leucyl-D-phenyllactyl-N-methyl-D-phenyllactyl-N-methyl-L-leucyl-D-milchsäure-tert.-butylester.
- EI-MS m/z (%):: 808 (M⁺,4); 680 (5), 624 (2); 462 (1); 348 (3); 259 (27); 100 (100)

### Beispiel 5

### N-Methyl-L-isoleucyl-D-lactyl-N-methyl-L-isoleucyl-D-lactyl-N-methyl-L-isoleucyl-D-milchsäure

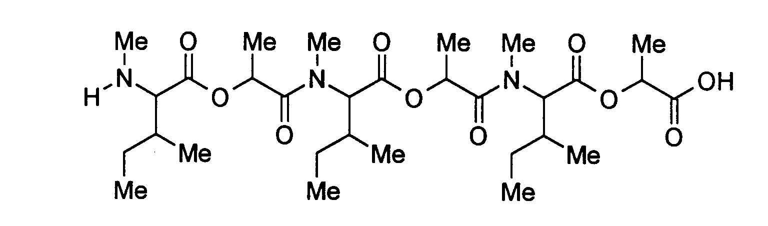

Die N-terminale Deblockierung erfolgt analog der Reaktionsvorschrift des Beispiels 4 innerhalb von ca. 2 Stunden unter Verwendung von:
1,00 g (1,33 mMol) N-Benzyloxycarbonyl-N-methyl-L-isoleucinyl-D-lactyl-N-methyl-L-isoleucyl-D-lactyl-N-methyl-L-isoleucyl-D-milchsäure,
0,15 g Pd(OH)₂/Kohle (20 % Pd-Gehalt),
20 ml Ethanol

Man erhält 0,81 g (100 % der Theorie) N-Methyl-L-isoleucyl-D-lactyl-N-methyl-L-isoleucyl-D-lactyl-N-methyl-L-isoleucyl-D-milchsäure.
- EI-MS m/z (%):: 615 (M⁺,3); 600 (1); 558 (7); 472 (8); 386 (14); 100 (100)

Analog können die in der nachstehenden Tabelle 1 aufgeführten Verbindungen der allgemeinen Formel (I; R⁷ bis R⁹ = -Me) als LDLDLD - Stereoisomere hergestellt werden.

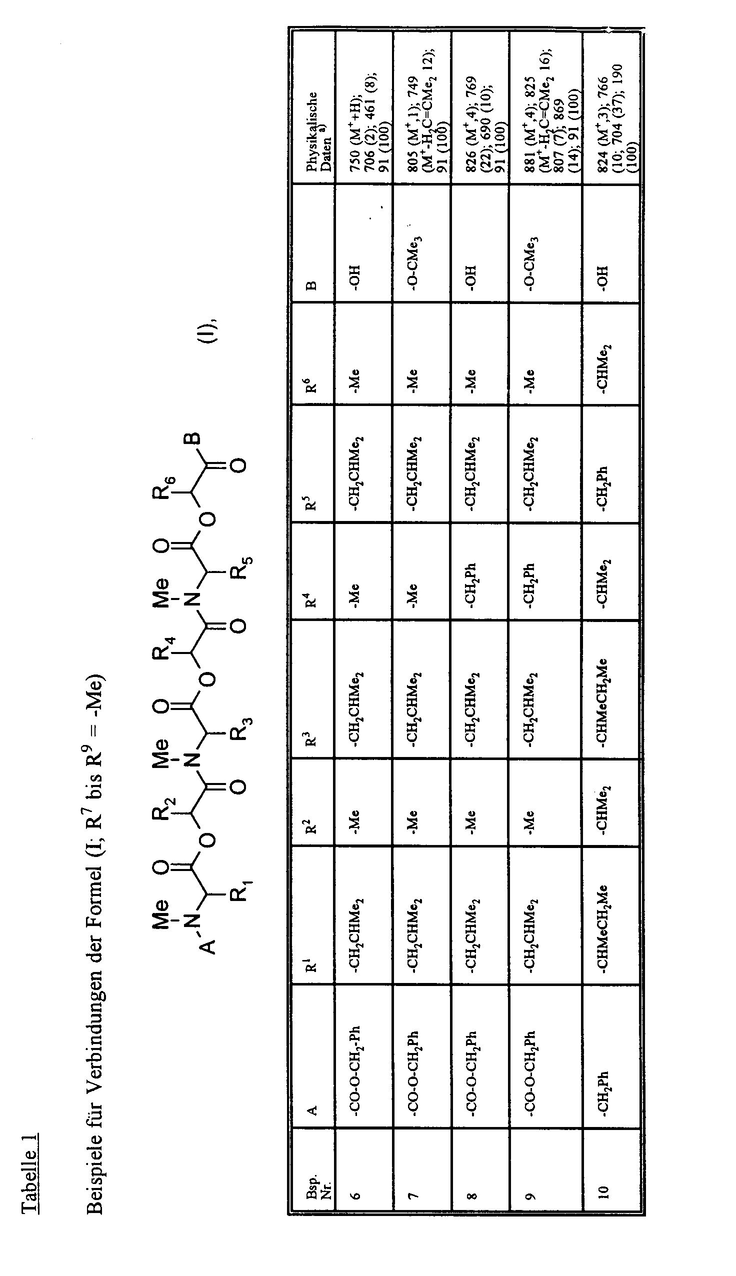

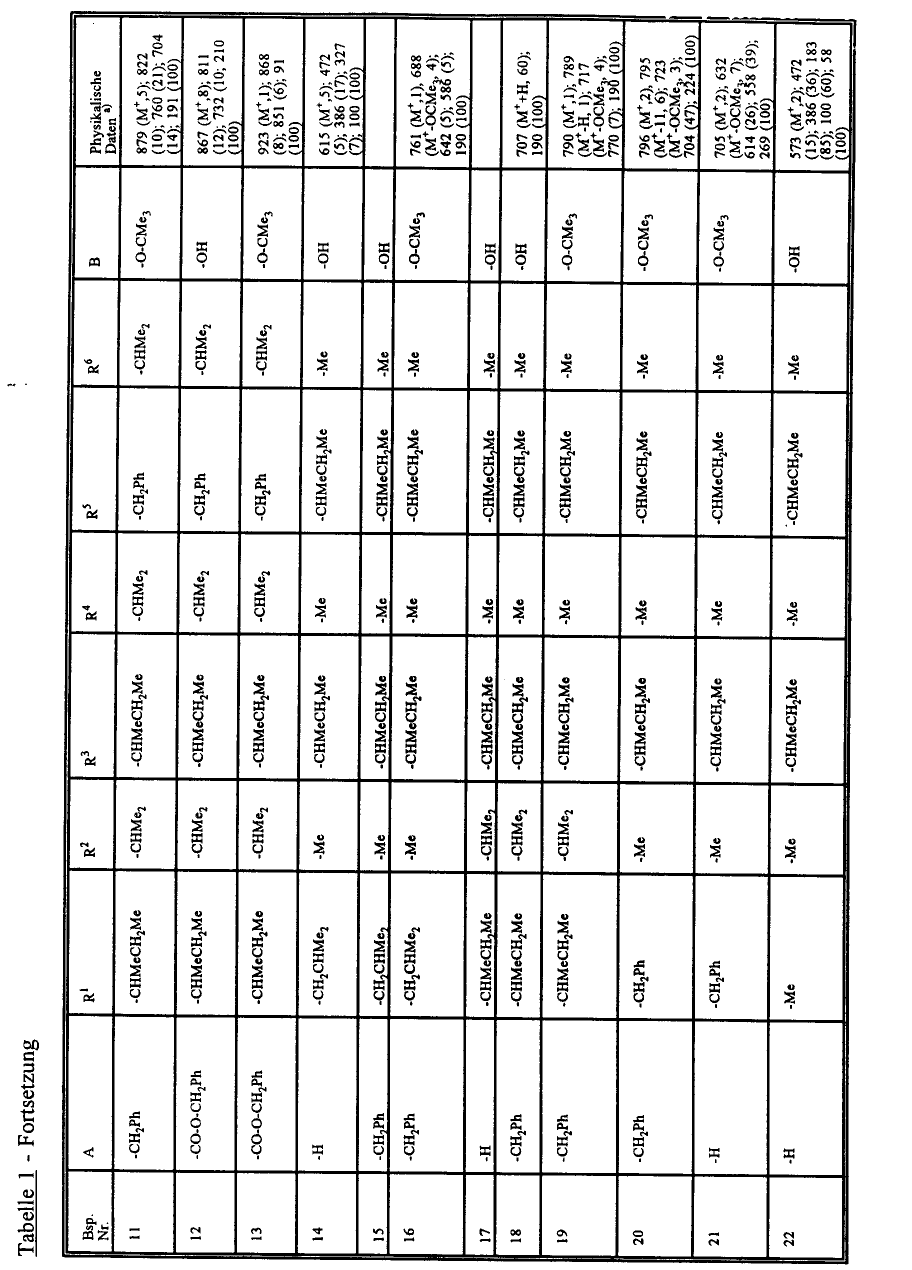

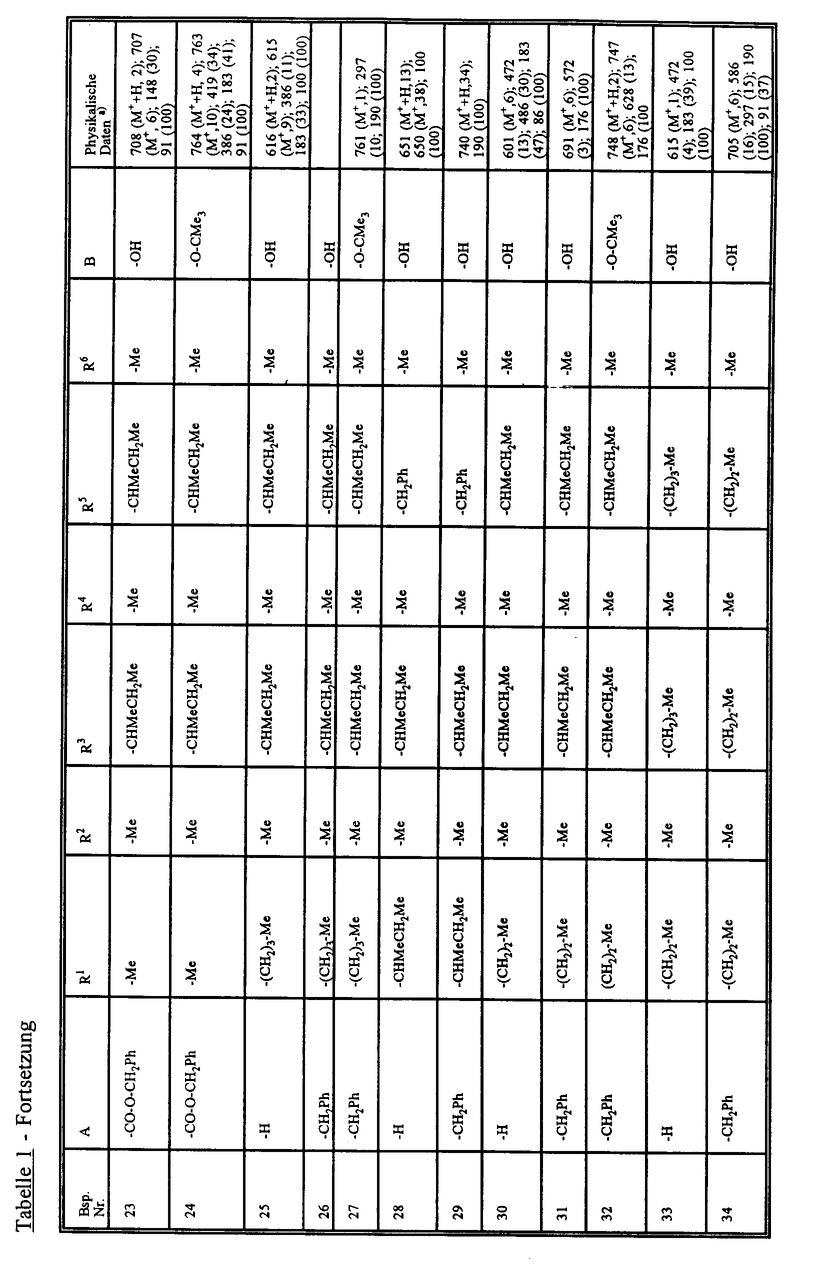

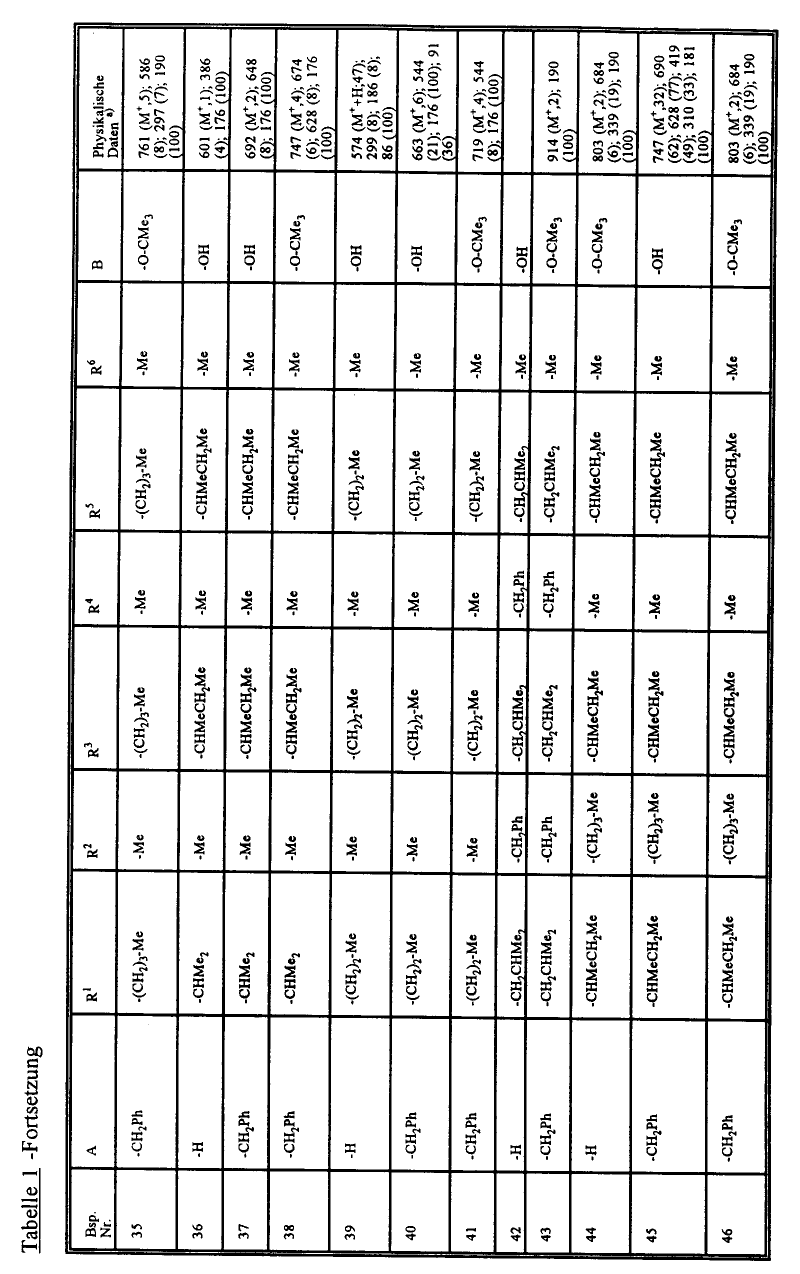

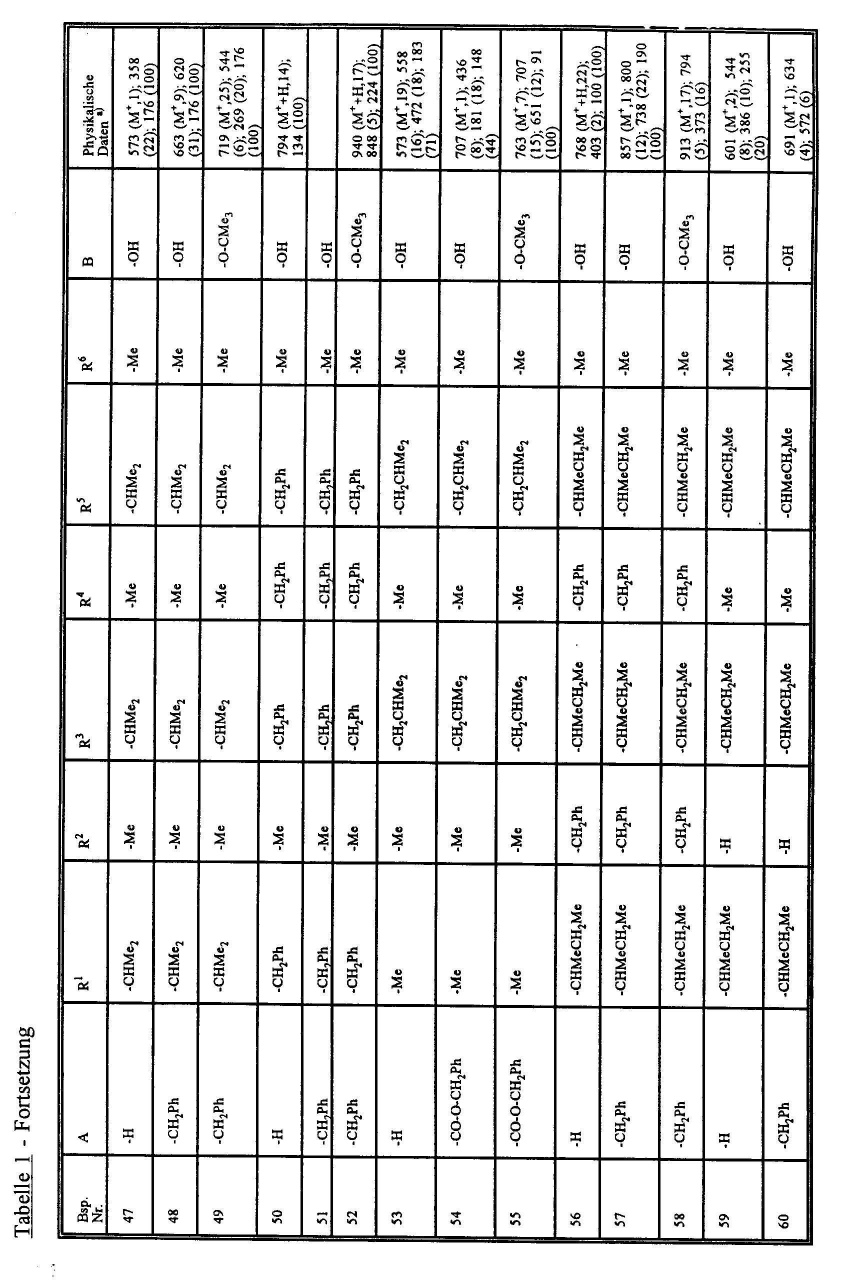

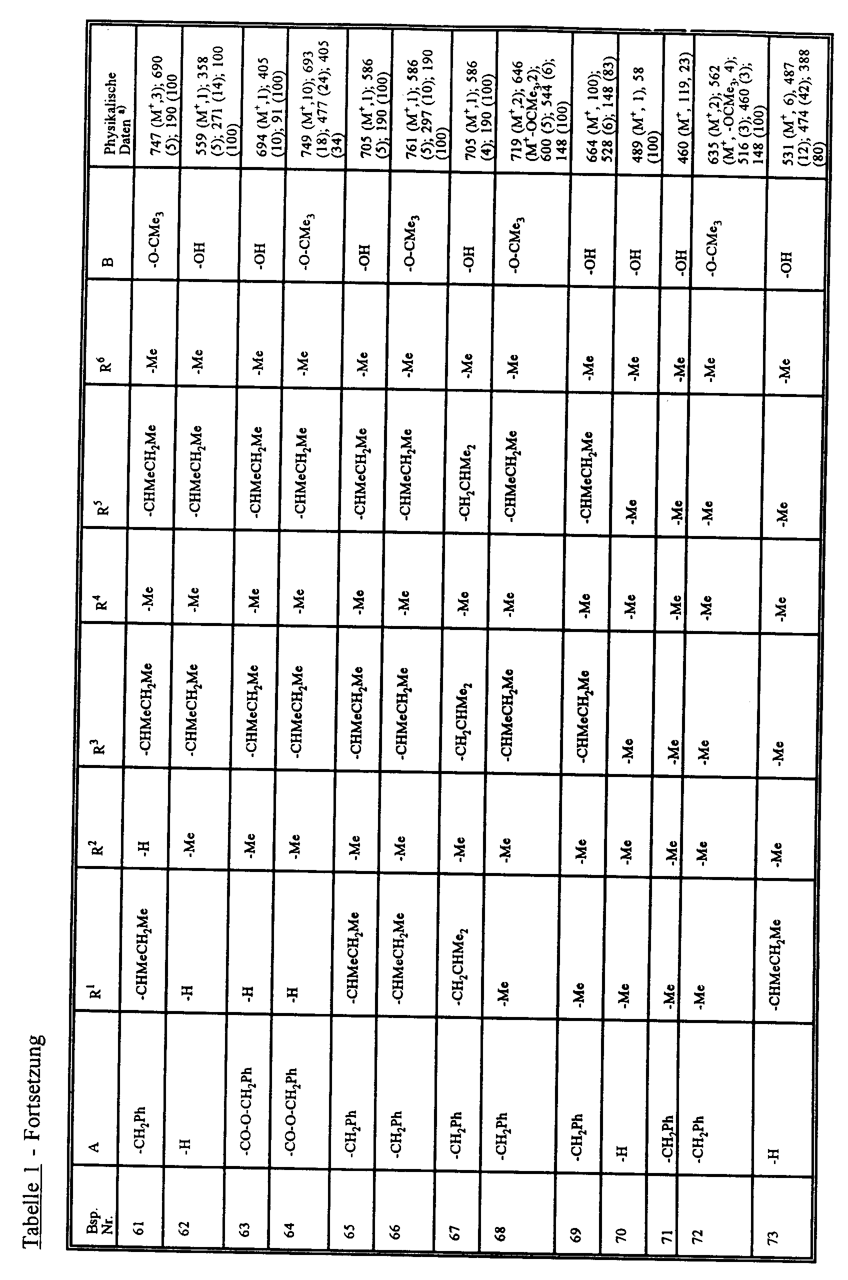

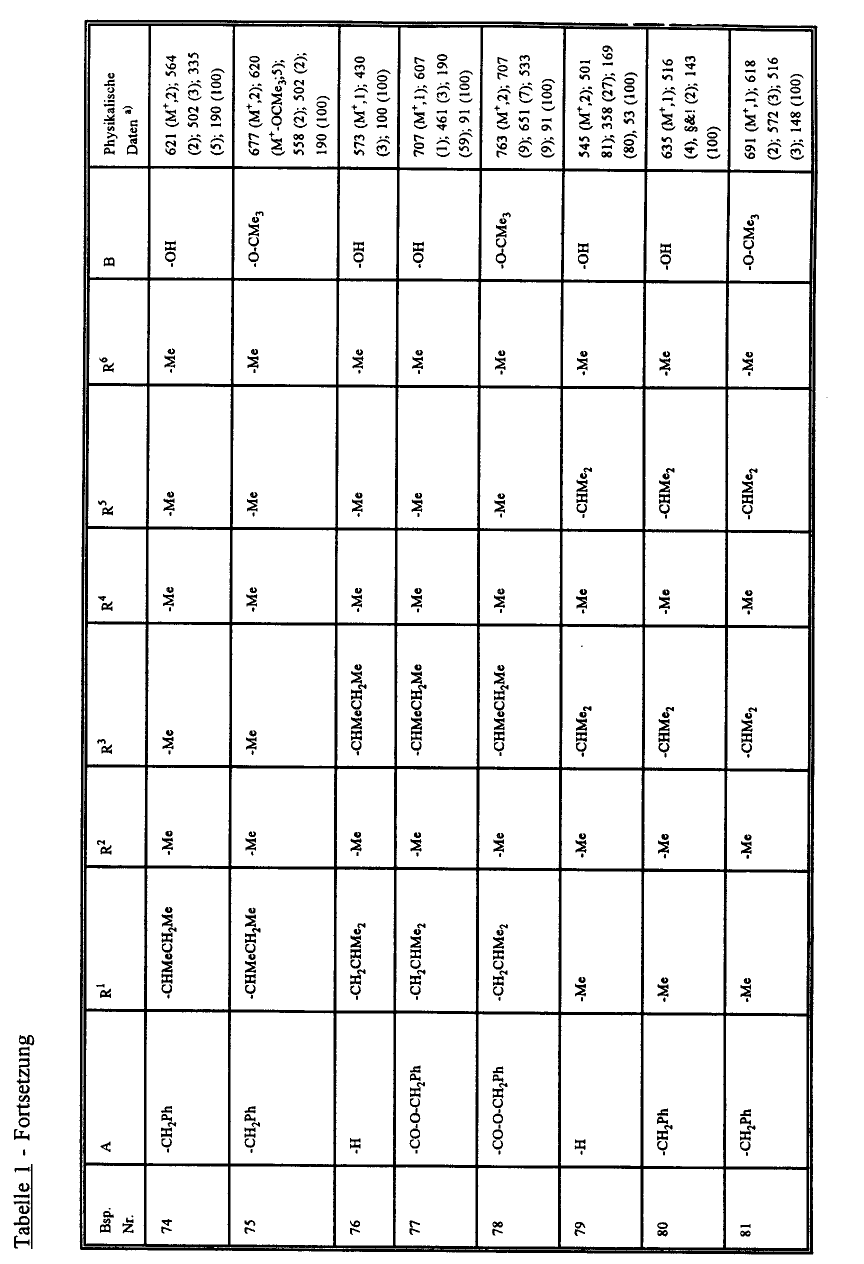

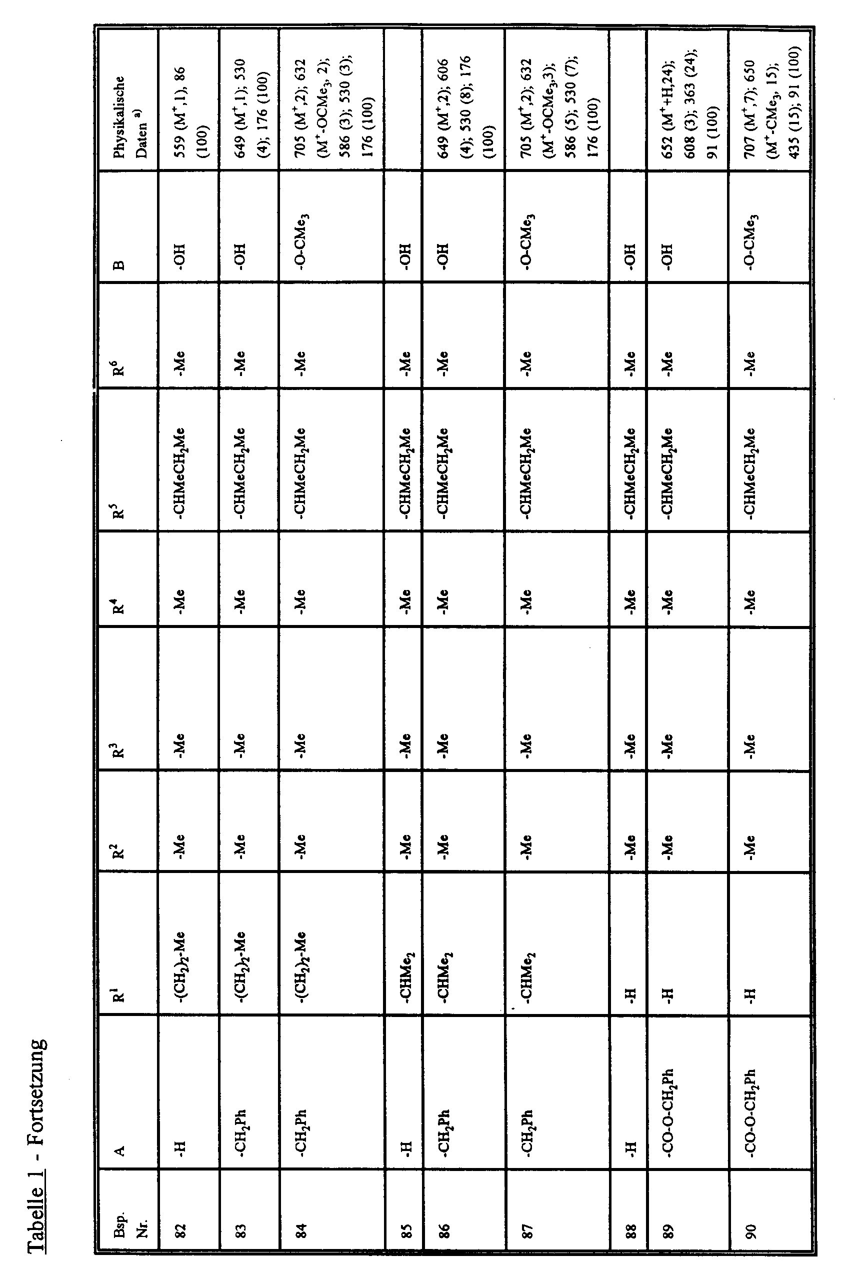

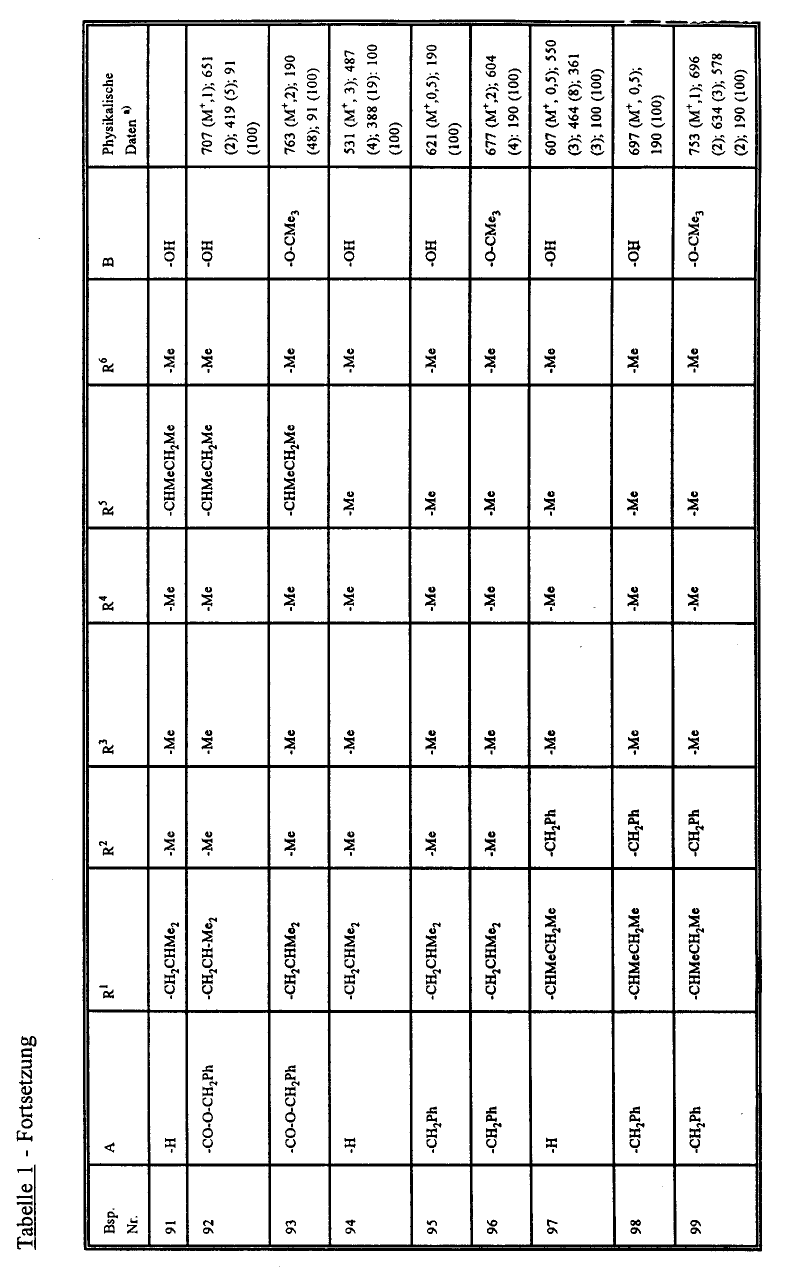

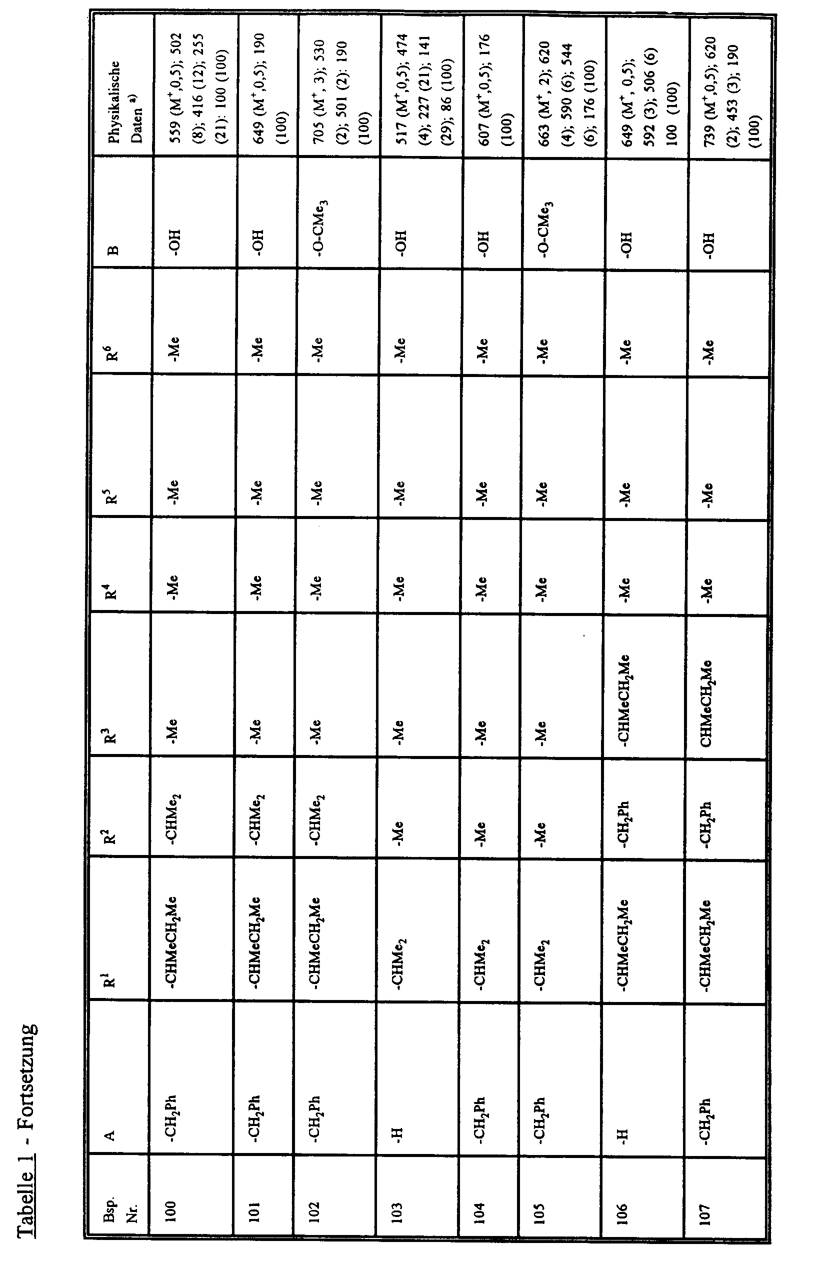

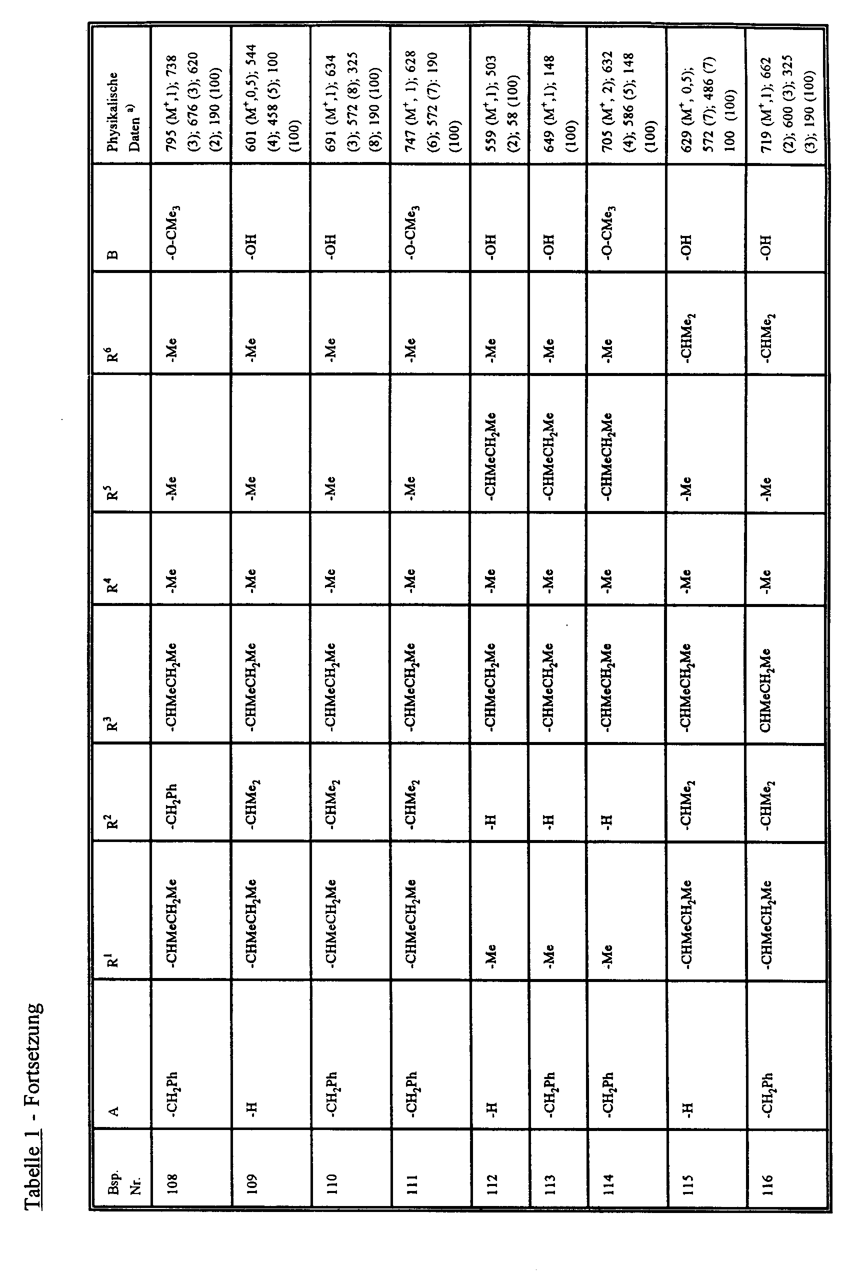

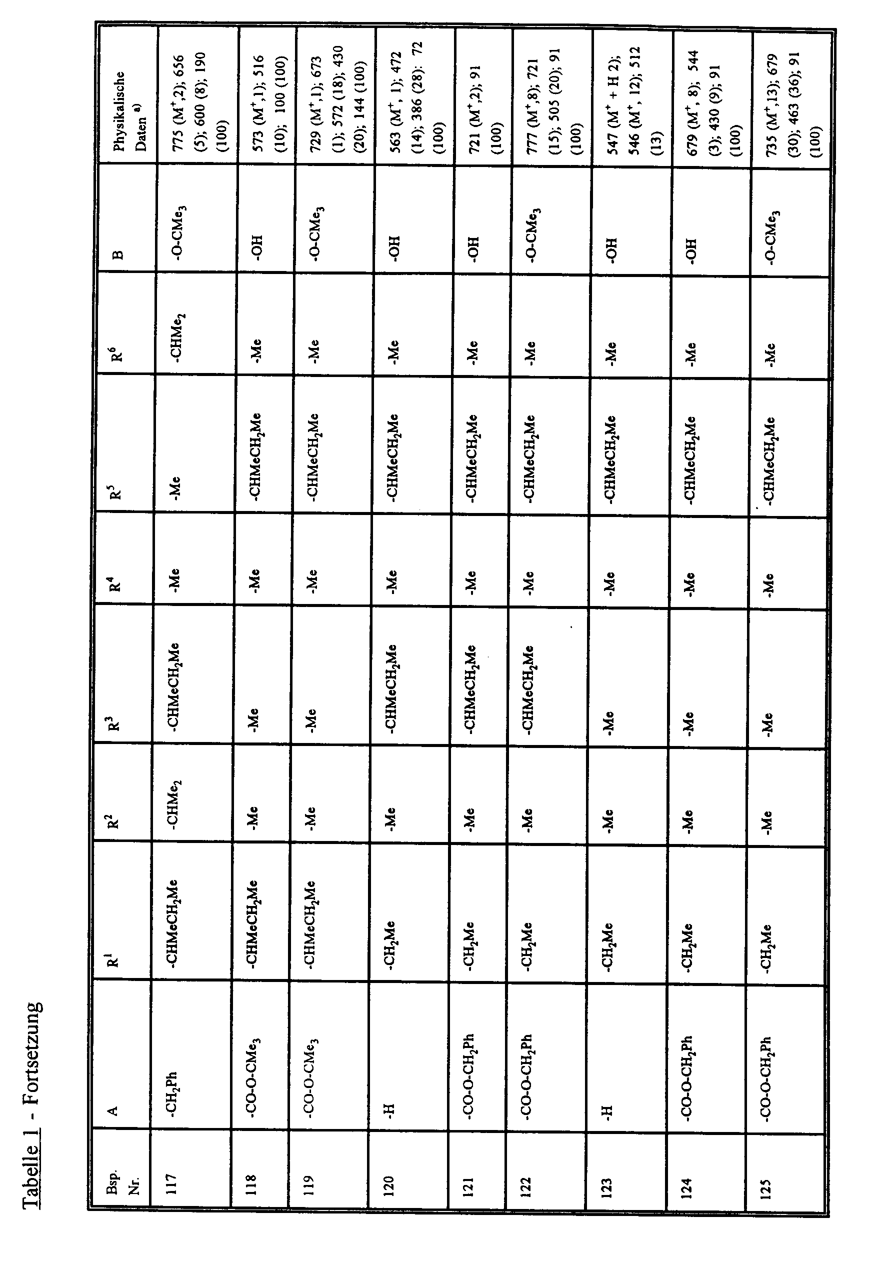

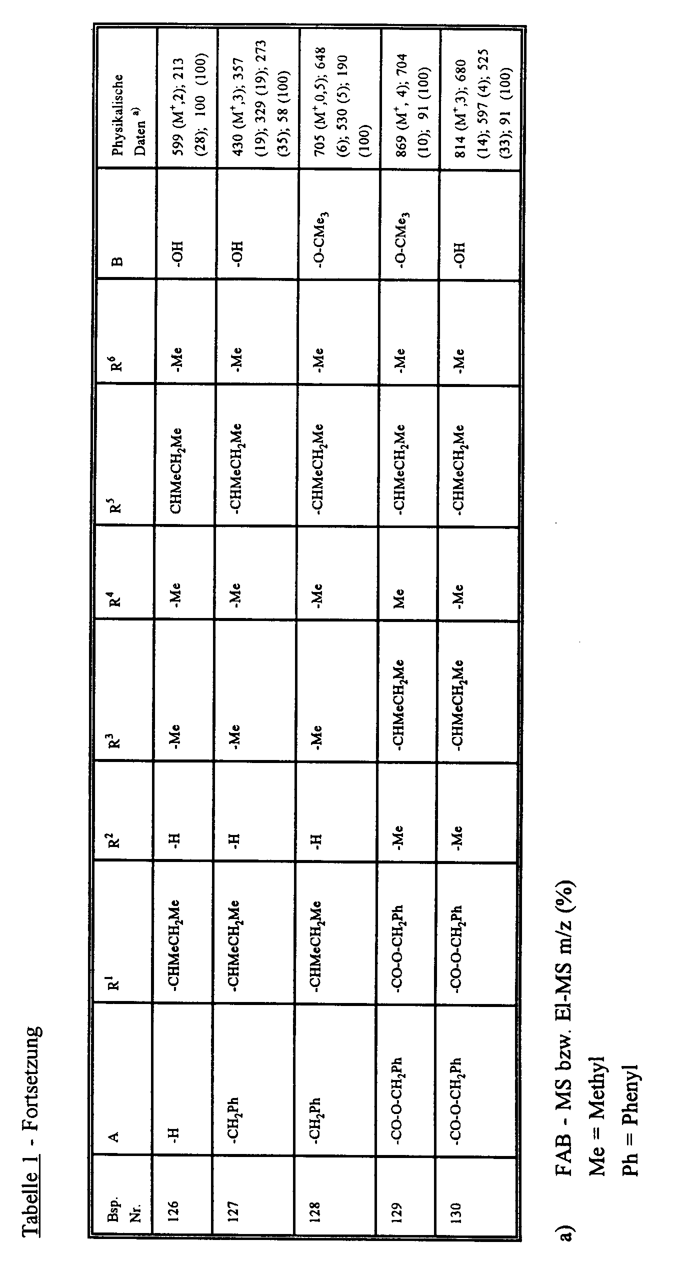

### Ausgangsstoffe der Formel (II) und (III)

### Beispiel ( II - 1 )

### N-Benzyl-N-methyl-L-leucyl-D-lactyl-N-methyl-L-leucyl-D-milchsäure-tert.-butylester

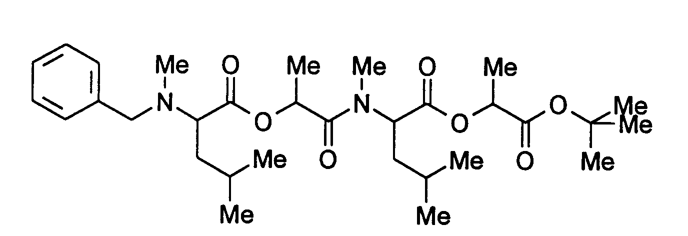

Die Kupplungsreaktion erfolgt analog der Reaktionsvorschrift des Beispiels 1 unter Verwendung von:
12,4 g (40,3 mMol) N-Benzyl-N-methyl-L-leucyl-D-milchsäure,
11,0 g (40,3 mMol) N-Methyl-L-leucyl-D-milchsäure-tert.-butylester,
100 ml Methylenchlorid,
11,5 g (88,7 mMol) N,N-Diisopropylethylamin ("Hünig's Base"),
11,3 g (44,3 mMol) Bis(2-oxo-3-oxazolidinyl)-phosphoniumsäurechlorid (BOP-Cl).

Das zurückbleibende Rohprodukt wird über eine Kieselgelsäule (Kieselgel 60 - Merck, Korngröße : 0,04 bis 0,063 mm) mit dem Fließmittel Toluol : Essigsäureethylester (20:1) chromatographiert. Man erhält 21,8 g (96,0 % der Theorie) N-Benzyl-N-methyl-L-leucyl-D-lactyl-N-methyl-L-leucyl-D-milchsäure-tert.-butylester.
- EI-MS m/z (%):: 562 (M⁺,3); 489 (M⁺- OCMe₃,7); 443 (2); 387 (3); 344 (1); 190 (PhCH₂-NMe-CH-CH₂Me₂,100); 120 (PhCH₂-NMe-,31)

### Beispiel (II-2)

### N-Benzyl-N-methyl-L-isoleucyl-D-lactyl-N-methyl-L-isoleucyl-D-milchsäure

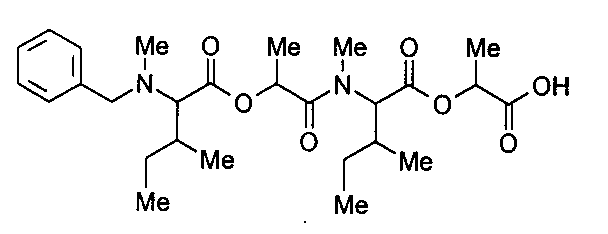

Die C-terminale Acidolyse erfolgt analog der Reaktionsvorschrift des Beispiels 3 unter Verwendung von:
- 9,0 g (16,0 mMol): N-Benzyl-N-methyl-L-isoleucyl-D-lactyl-N-methyl-L-isoleucyl-D-milchsäure-tert.-butylester
- 300 ml: Methylenchlorid.

Man erhält 8,2 g (100 % der Theorie) N-Benzyl-N-methyl-L-isoleucyl-D-lactyl-N-methyl-L-isoleucyl-D-milchsäure.
- EI-MS m/z (%):: 506 (M⁺,3); 449 (7); 190 (PhCH₂-NMe-CH-CHMeCH₂Me, 100)

### Beispiel (III-1)

### N-Methyl-L-leucyl-D-lactyl-N-methyl-L-leucyl-D-milchsäure-tert.-butylester

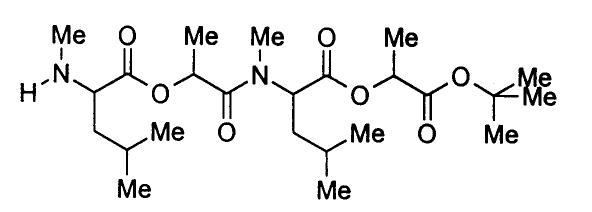

21,8 g (38,7 mMol) Bn-(-L-MeLeu-D-Lac-)₂-O-^{t}Bu werden in 300 ml Ethanol gelöst und in Gegenwart von 2,2 g Pd(OH)₂/Kohle [20 % Pd-Gehalt] bis zur Beendigung der Wasserstoffaufnahme hydriert (ca. 2,5 Stunden). Nach Abfiltrieren des Katalysators wird die gesamte Reaktionslösung im Vakuum eingeengt. Man erhält 18,3 g (100 % der Theorie) N-Methyl-L-leucyl-D-lactyl-N-methyl-L-leucyl-D-milchsäure-tert.-butylester, der ohne weitere Reinigung für die Kupplungsreaktion verwendet werden kann.
- EI-MS m/z (%):: 472 (M⁺,4); 457 (1); 428 (1); 399 (6); 100 (HNMe-CH-CH₂Me₂,100)

Analog können die in der nachstehenden Tabelle 2 aufgeführten Verbindungen der allgemeinen Formel (III; R⁸, R⁹ = Me) als LDLD-Stereoisomere hergestellt werden.

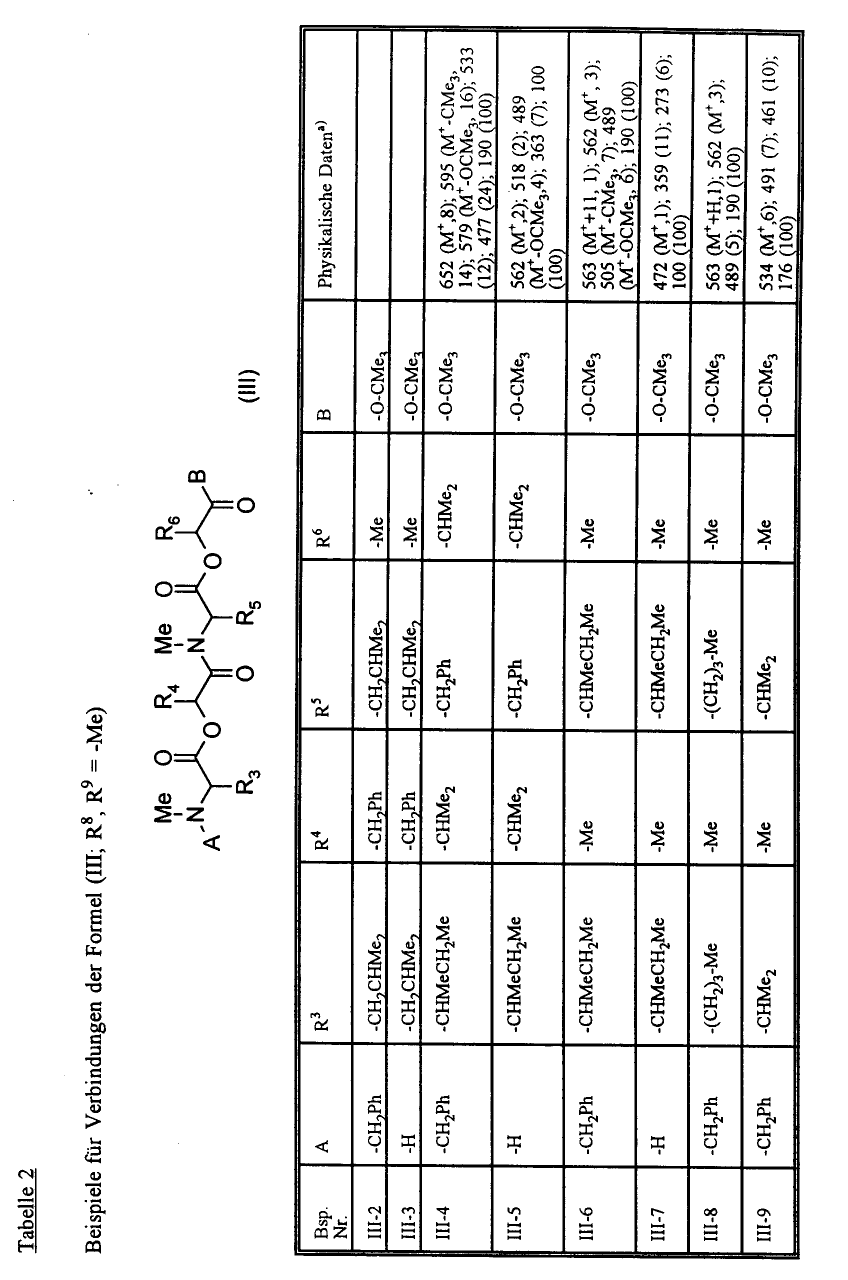

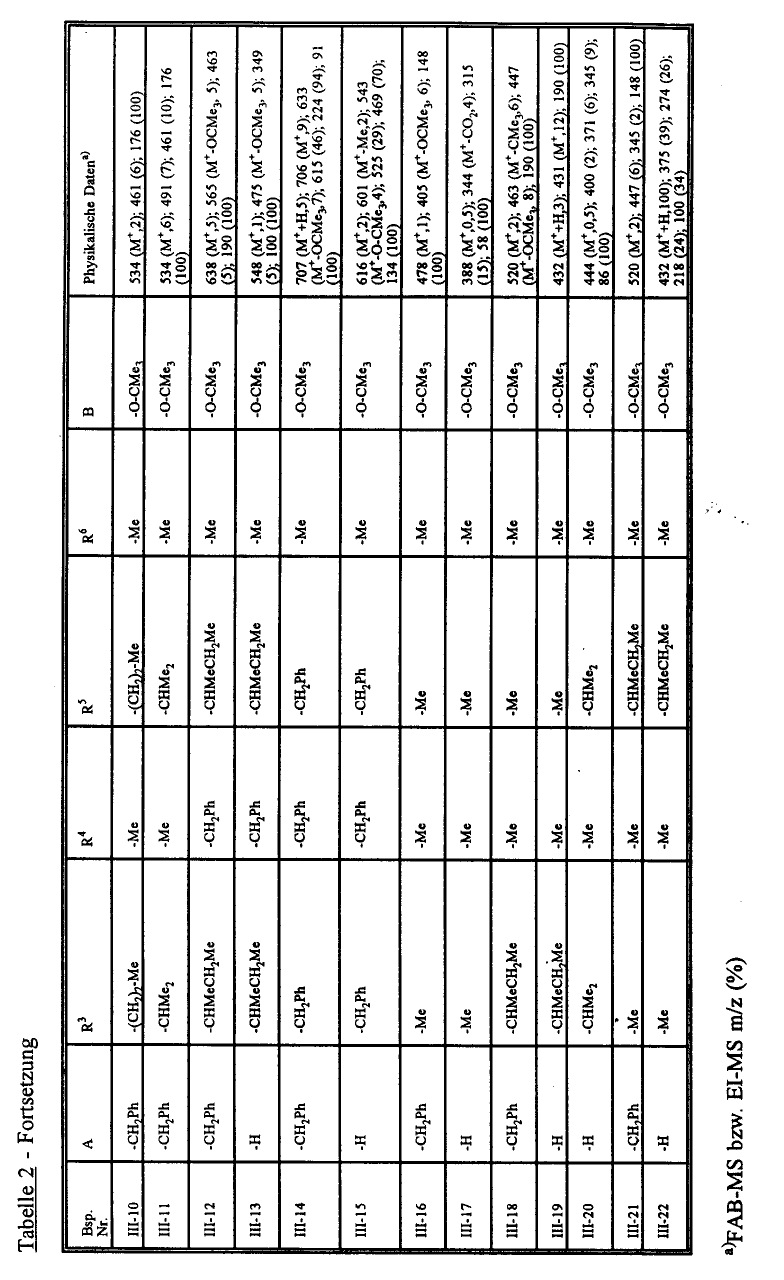

### Ausgangsstoffe der Formel ( IV ), ( V ) und ( VI )

### Beispiel ( IV - 1 )

### N-Benzyloxycarbonyl-N-methyl-L-leucyl-D-milchsäure-tert.-butylester

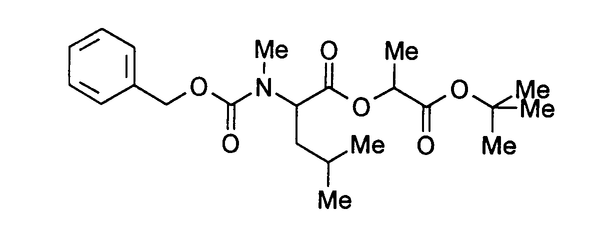

10,0 g (35,8 mMol) N-Benzyloxycarbonyl-N-methyl-L-leucin werden in 150 ml Methanol und 15 ml Wasser gelöst, mit 19,5 ml einer 20 %igen Cäsiumcarbonat-Lösung versetzt und ca. eine Stunde bei Raumtemperatur gerührt. Anschließend wird zweimal mit ca. 50 ml absolutem Dimethylformamid versetzt, im Vakuum eingeengt und im Hochvakuum getrocknet. Das Cäsiumsalz wird in 75 ml Dimethylformamid vorgelegt, mit 7,0 g (35,8 mMol) L-2-Chlor-propionsäure-tert.-butylester versetzt und ca. 18 Stunden bei Raumtemperatur gerührt. Die gesamte Reaktionslösung wird im Vakuum eingeengt, der ölige Rückstand in Methylenchlorid aufgenommen und zweimal mit Wasser geschüttelt. Danach wird die organische Phase abgetrennt, über Natriumsulfat getrocknet und im Vakuum eingeengt.

Das zurückbleibende Rohprodukt wird über eine Kieselgelsäule (Kieselgel 60 - Merck, Korngröße : 0,04 bis 0,063 mm) mit dem Fließmittel Toluol : Essigsäureethylester (40:1) chromatographiert. Man erhält 14,4 g (100 % der Theorie) N-Benzyloxycarbonyl-N-methyl-L-leucyl-D-milchsäure-tert.-butylester.
- EI-MS m/z (%):: 407 (M⁺,2); 351 (10); 234 (39); 387 (3); 344 (1); 190 (PhCH₂-NMe-CH-CH₂Me₂,69); 91 (PhCH₂, 100)

### Beispiel ( V - 1 )

### N-Benzyl-N-methyl-L-isoleucyl-D-hydroxyisovaleriansäure

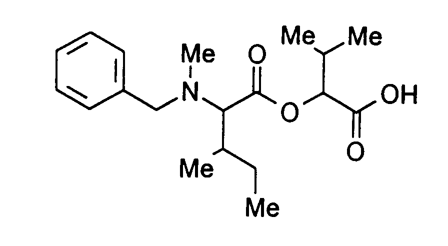

Die C-terminale Acidolyse erfolgt analog der Reaktionsvorschrift des Beispiels (I-2) unter Verwendung von:
- 10,5 g (26,8 mMol): N-Benzyl-N-methyl-L-isoleucyl-D-hydroxyisovaleriansäure-tert.-butylester,
- 250 ml: Methylenchlorid.

Man erhält 8,5 g (94,5 % der Theorie) N-Benzyl-N-methyl-L-isoleucyl-D-hydroxyisovaleriansäure, die ohne weitere Reinigung weiter umgesetzt werden kann.
- EI-MS m/z (%):: 335 (M⁺,1); 278 (19); 190 (PhCH₂-NMe-CH-CHMeCH₂Me,100); 91 (PhCH₂,84)

### Beispiel ( VI - 1 )

### N-Methyl-L-phenylalanyl-D-hydoxyvaleriansäure-tert.-butylester

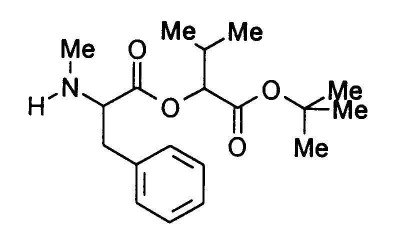

Die N-terminale Deblockierung erfolgt analog der Reaktionsvorschrift des Beispiels ( III - 2 ) unter Verwendung von:
10,0 g (23,5 mMol) N-Benzyl-N-methyl-L-phenylalanyl-D-hydroxyisovaleriansäure-tert.-butylester,
250 ml Ethanol,
1,0 g Pd(OH)₂/Kohle [20 % Pd-Gehalt]

Man erhält 7,5 g (95,2 % der Theorie) N-Methyl-L-phenylalanyl-D-hydroxyvaleriansäure-tert.-butylester, die ohne weitere Reinigung weiter umgesetzt werden kann.
¹H-NMR (400 MHz, CDCl₃, δ): 0,80; 0,85 (2d, 6H, 2 x -CH₃; J = 6,9 Hz); 1,46 (s, 9H, -C(CH₃)₃); 2,42 (s, 3H, -N-CH₃); 2,94; 2,97 (2d, 2H, - CH₂-Phe); 3,55 (m, 1H,-O-CH); 4,58 (d, 1H, -N-CH; J = 4,7 Hz); 7,18-7,26 (m, 5H, arom.-H) ppm
   - EI-MS m/z (%):: 336 (M⁺+ H,7); 335 (M⁺,2); 262 (M⁺- O-CMe₃,12); 188 (100); 134 (81)

Analog können die in den nachstehenden Tabellen 3, 4 und 5 aufgeführten Verbindungen der allgemeinen Formeln (IV; R⁷ = Me), (V; R⁸ = -Me) und (VI; R⁹ = -Me) als L-D-Stereoisomere hergestellt werden.

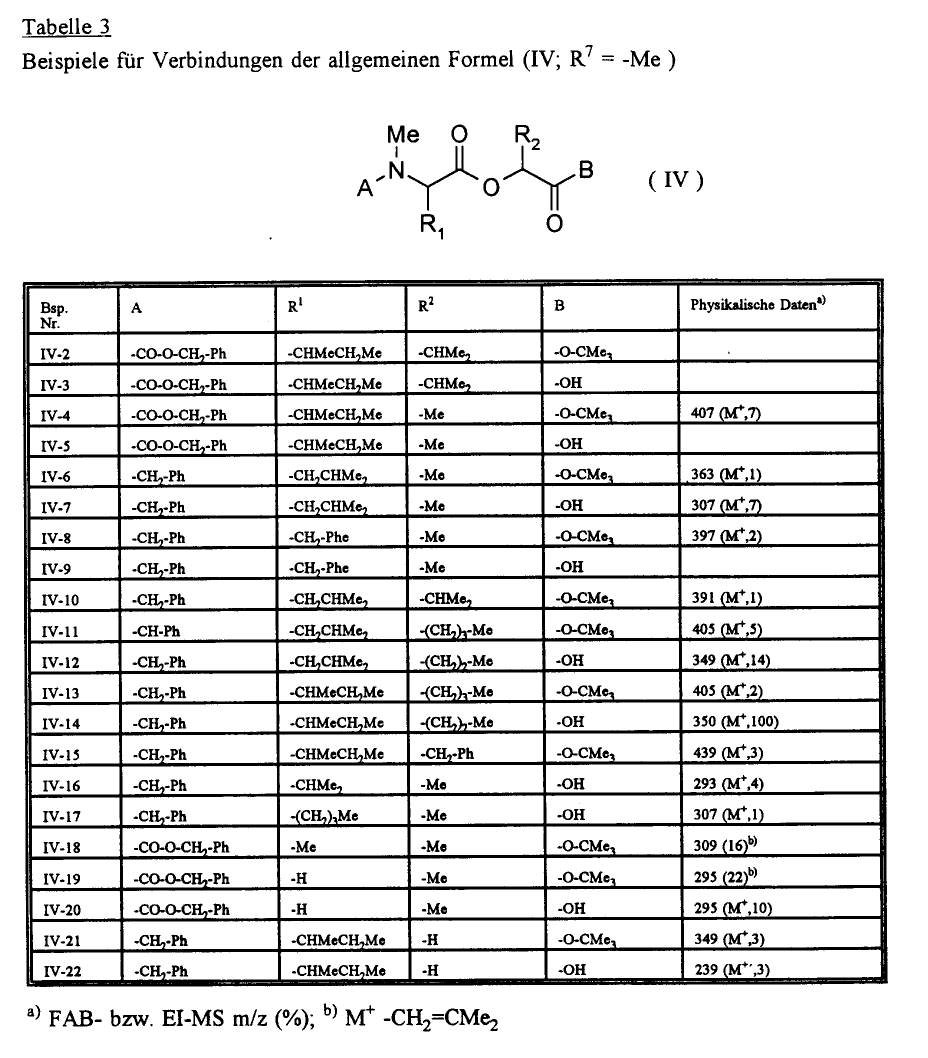

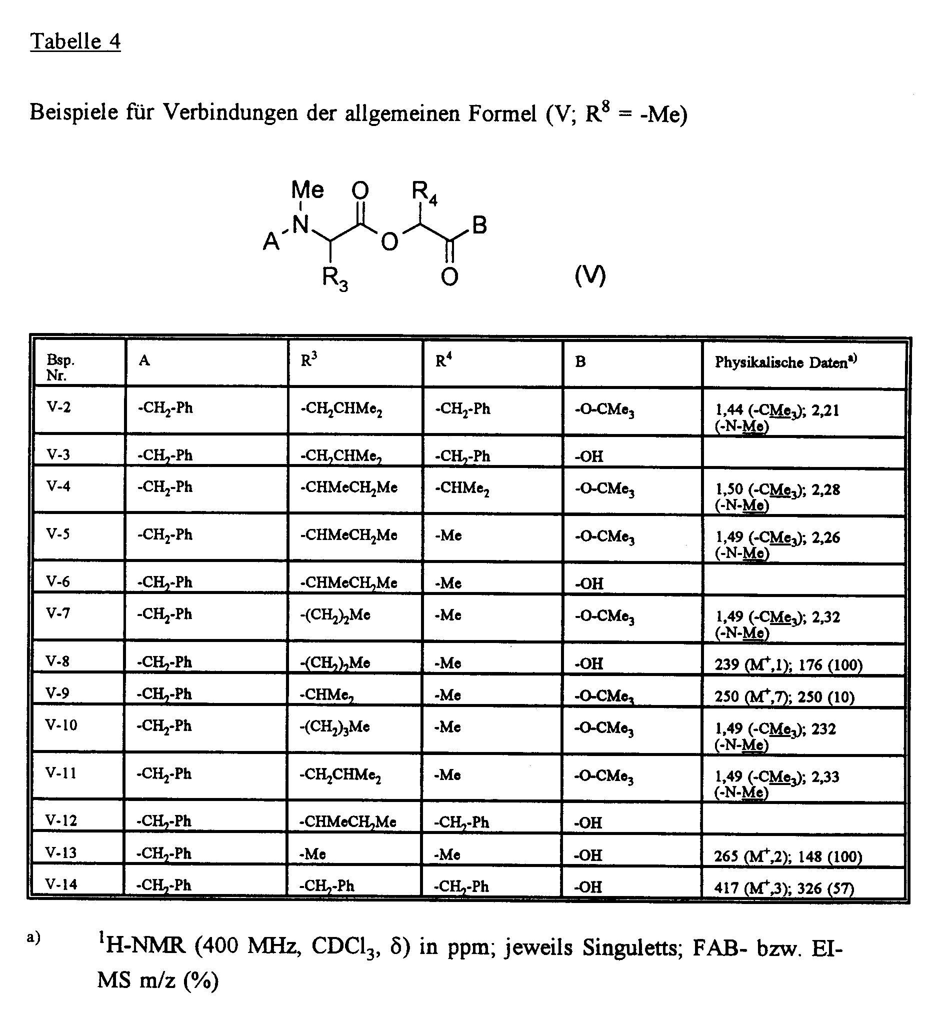

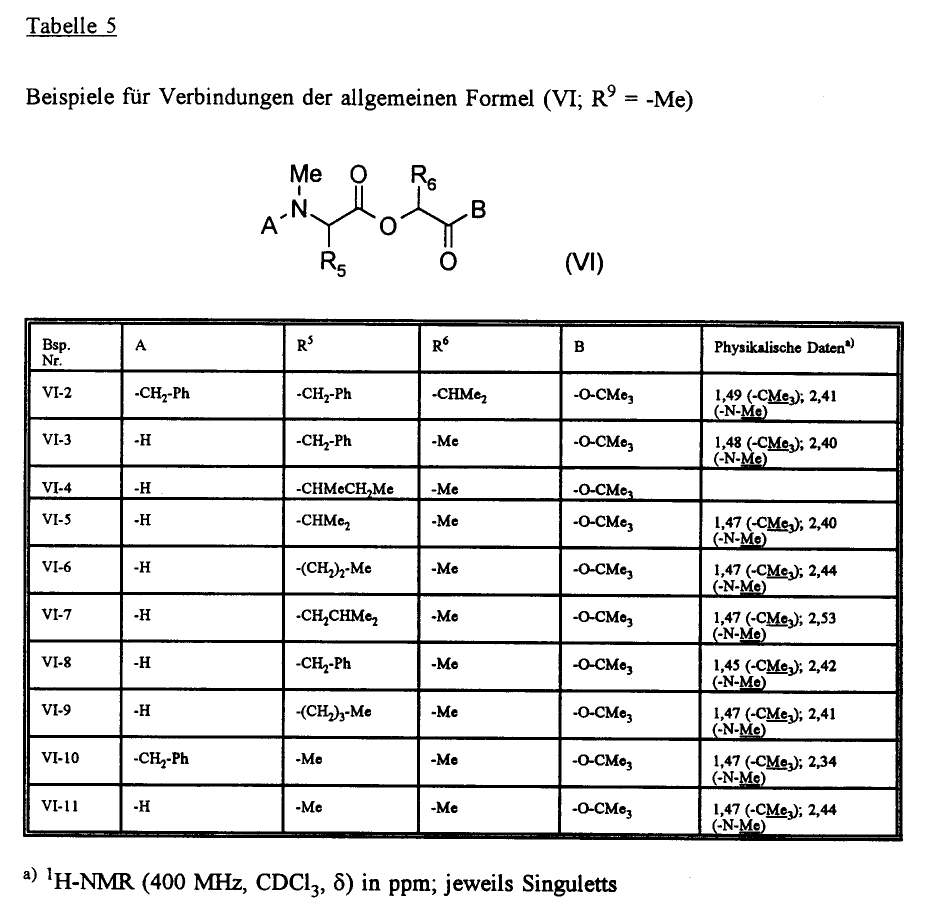

## Patentansprüche

1. Verwendung von offenkettigen Hexadepsipeptiden der allgemeinen Formel (I) 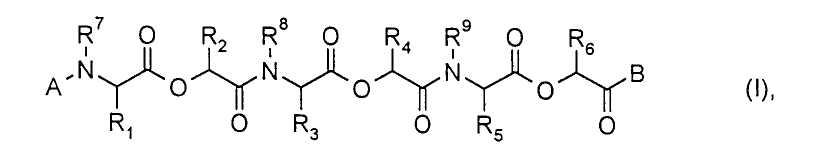
A für Wasserstoff, Alkyl, Aralkyl oder einem Arylrest, insbesondere für einen Rest der Formel -CO-R¹⁰ steht, worin
R¹⁰ für geradkettiges, verzweigtes Alkyl, Aralkyl, Alkoxy oder Arylalkoxy mit bis zu 6 Kohlenstoffatomen im Alkylteil steht,
R¹, R³ und R⁵ unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylallryl, gegebenenfalls substituiertes Aryl, Hetarylmethyl sowie Arylalkyl, wobei als Substituenten genannt seien Halogen, Hydroxy, Alkyl, Alkoxy, Nitro oder ein Rest -NR¹¹R¹², wobei R¹¹ und R¹² unabhängig voneinander für Wasserstoff oder Alkyl stehen oder R¹¹R¹² gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch C₁-C₄-Alkyl substituiert ist,
R², R⁴ und R⁶ unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Alkylthioalkyl, Alkylsulfinylalkyl, Alkysulfonylalkyl, Carboxyalkyl, Alkoxycarbonylalkyl, Arylalkoxycarbonylalkyl, Carbamoylalkyl, Aminoalkyl, Alkylaminoalkyl, Dialkcylaminoalkyl, Alkoxycarbonylaminoalkyl, Alkenyl, Cycloalkyl, Cycloalkylalkyl, Hetarylmethyl sowie gegebenenfalls substituiertes Arylalkyl, wobei als Substituenten genannt seien Halogen, Hydroxy, Alkyl, Alkoxy, Nitro oder ein Rest -NR¹¹R¹², wobei R¹¹ und R¹² unabhängig voneinander für Wasserstoff oder Alkyl stehen oder R¹¹R¹² gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch C₁-C₄-Alkyl substituiert ist,
R⁷, R⁸ und R⁹ unabhängig voneinander für Wasserstoff, C₁₋₈-Alkyl, C₃₋₆-Gycloalkyl oder für Aralkyl stehen,
B für Hydroxy oder Alkoxy mit bis zu 4 Kohlenstoffatomen steht,
sowie deren optische Isomere und Racemate,
zur Herstellung von endoparasitiziden Mitteln für Medizin und Tiermedizin.

2. Endoparasitzide Mittel, gekennzeichnet durch einen Gehalt an mindestens einem offenkettigen Hexadepsipeptid der Formel (I) gemäß Anspruch 1.

3. Verfahren zur Herstellung von endoparasitiziden Mitteln, dadurch gekennzeichnet, daß man offenkettige Hexadepsipeptide der Formel (I) gemäß Anspruch 1 mit Streckmitteln und/oder oberflächenakfiven Mitteln vermischt.

4. Verwendung von offenkettigen Hexadepsipeptiden der Formel (I) gemäß Anspruch 1
in welcher
A für Wasserstoff, C₁₋₄-Alkyl, Benzyl oder für eine Gruppe der Formel -CO-R¹⁰ steht,
worin
R¹⁰ für geradkettiges oder verzweigtes C₁₋₄-Alkyl, C₁₋₄-Alkoxy oder Phenylalkoxy mit bis zu 6 Kohlenstoffatomen im Alkylteil steht,
R¹, R³ und R⁵ unabhängig voneinander für Wasserstoff, geradkettiges oder verzweigtes C₁-C₈-Alkyl, Hydroxy-C₁-C₆-alkyl, C₁-C₄-Alkanoyloxy-C₁-C₆alkyl, C₁-C₄-Alkoxy-C₁-C₆-alkyl, Aryl-C₁-C₄-alkyloxy-C₁-C₆-alkyl, Mercapto-C₁-C₆-alkyl, C₁-C₄-Alkylthio-C₁-C₆-alkyl, C₁-C₄-Alkylsulfinyl-C₁-C₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁-C₄-Arylalkoxycarbonyl-C₁-C₆-alkyl, Carbamoyl-C₁-C₆-alkyl, Amino-C₁-C₆-alkyl, C₁-C₄-Alkylamino-C₁-C₆-alkyl, C₁-C₄-Dialkylamino-C₁-C₆-alkyl, Guanido-C₁-C₆-alkyl, C₁-C₄-Alkoxycarbonylamino-C₁-C₆-alkyl, 9-Fluorenylmethoxycarbonyl(Fmoc)aminopropyl, 9-Fluorenylmethoxy-carbonyl(Fmoc)aminobutyl, C₂-C₈-Alkenyl, c₃-c₇-Cycloalkyl, C₃-C₇-Cycloalkyl-C₁-C₄-alkyl, Phenyl, Benzo[b]thien-1-yl-methyl, Benzo[b]thien-3-yl-methyl, Naphth-1-yl-methyl, Naphth-2-yl-methyl, Pyrid-2-yl-methyl, Pyrid-3-yl-methyl, Fur-2-yl-methyl, Fur-3-yl-methyl, Thien-2-yl-methyl, Thien-3-yl-methyl, Indol-3-yl-methyl, N-Methyl-indol-3-yl-methyl, Imidazol-4-yl-methyl, N-Methyl-imidazol-4-yl-methyl, Phenyl-C₁-C₄-alkyl, die gegebenenfalls durch Reste aus der Reihe Halogen, Hydroxy, C₁-C₄-Alkyl, C₁-C₄-Alkoxy, Methylendioxy oder Ethylendioxy, Nitro oder C₁-C₄-Alkylamino, C₁-C₄-Dialkylamino, C₃-C₆-Cycloalkylamino, C₃-C₆-Cycloalkoxyamino, C₃-C₆-Cycloalkylthioxyamino substituiert sein können,
R², R⁴ und R⁶ unabhängig voneinander für Wasserstoff, geradkettiges oder verzweigtes C₁-C₈-Alkyl, Hydroxy-C₁-C₆-alkyl, C₁-C₄-Alkanoyloxy-C₁-C₆-alkyl, C₁-C₄-Alkoxy-C₁-C₆-alkyl, Aryl-C₁-C₄-alkyloxy-C₁-C₆-alkyl, Mercapto-C₁-C₆-alkyl, C₁-C₄-Alkylthio-C₁-C₆-alkyl, C₁-C₄-Alkylsulfinyl-C₁-C₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁-C₄-Arylalkoxycarbonyl-C₁-C₆-alkyl, Carbamoyl-C₁-C₆-alkyl, Amino-C₁-C₆-alkyl, C₁-C₄-Alkylamino-C₁-C₆-alkyl, C₁-C₄-Dialkylamino-C₁-C₆-alkyl, Guanido-C₁-C₆-alkyl, C₁-C₄-Alkoxycarbonylamino-C₁-C₆-alkyl, 9-Fluorenylmethoxycarbonyl(Fmoc)aminopropyl, 9-Fluorenylmethoxycarbonyl(Fmoc)aminobutyl, C₂-C₈-Alkenyl, C₃-C₇-Cycloalkyl, C₃-C₇-Cycloalkyl-C₁-C₄-alkyl, Phenyl, Benzo[b]thien-1-yl-methyl, Benzo[b]thien-3-yl-methyl, Naphth-1-yl-methyl, Naphth-2-yl-methyl, Pyrid-2-yl-methyl, Pyrid-3-yl-methyl, Fur-2-yl-methyl, Fur-3-yl-methyl, Thien-2-yl-methyl, Thien-3yl-methyl, Indol-3-yl-methyl, N-Methyl-indol-3-yl-methyl, Imidazol-4-yl-methyl, N-Methyl-imidazol-4-yl-methyl, Phenyl-C₁-C₄-alkyl, das gegebenenfalls durch Reste aus der Reihe Halogen, Hydroxy, C₁-C₄-Alkyl, C₁-C₄-Alkoxy, Nitro oder C₁-C₄-Alkylamino, C₁-C₄-Dialkylamino, C₃-C₆-Cycloalkylamino, C₃-C₆-Cycloalkoxyamino, C₃-C₆-Cycloalkylthioxyamino substituiert sein kann,
R⁷, R⁸ und R⁹ unabhängig voneinander für Wasserstoff, geradkettiges oder verzweigtes C₁-C₄-Alkyl stehen,
B für Hydroxy oder tert.-Butyloxy steht,
sowie deren optische Isomere und Racemate.

5. Verwendung von offenkettigen Hexadepsipeptiden der Formel (I) gemäß Anspruch 1
in welcher
A für Wasserstoff oder Benzyl steht,
oder für eine Gruppe der Formel -CO-R¹⁰ steht,
worin
R¹⁰ für geradkettiges oder verzweigtes Alkoxy oder Arylalkoxy mit bis zu 6 Kohlenstoffatomen im Alkylteil steht,
R¹, R³ und R⁵ unabhängig voneinander für Wasserstoff, geradkettiges oder verzweigtes C₁-C₈-Alkyl, C₁-C₄-Alkanoyloxy-C₁-C₆-alkyl, C₁-C₄-Alkoxy-C₁-C₆-alkyl, Aryl-C₁-C₄-alkyloxy-C₁-C₆-alkyl, C₁-C₄-Alkoxycarbonylamino-C₁-C₆-alkyl, C₂-C₈-Alkenyl, C₃-C₇-Cycloalkyl, C₃-C₇-Cycloalkyl-C₁-C₄-alkyl, Phenyl, Naphth-1-yl-methyl, Pyrid-2-yl-methyl, Pyrid-3-yl-methyl, Thien-2-yl-methyl, Thien-3-yl-methyl, Indol-3-yl-methyl, N-Methyl-indol-3-yl-methyl, Imidazol-4-yl-methyl, N-Methyl-imidazol-4-yl-methyl, Phenyl-C₁-C₄-alkyl, das gegebenenfalls durch einen oder mehrere gleiche oder verschiedene der in Anspruch 4 angegebenen Reste substituiert sein kann,
R², R⁴ und R⁶ unabhängig voneinander für Wasserstoff, geradkettiges oder verzweigtes C₁-C₈-Alkyl, C₁-C₄-Alkanoyloxy-C₁-C₆-alkyl, C₁-C₄-Alkoxy-C₁-C₆-alkyl, Aryl-C₁-C₄-alkyloxy-C₁-C₆-alkyl, C₁-C₄-Alkoxycarbonylamino-C₁-C₆-alkyl, C₂-C₈-Alkenyl, C₃-C₇-Cycloalkyl, C₃-C₇-Cycloalkyl-C₁-C₄-alkyl, Phenyl, Benzo[b]thien-2-yl-methyl, Naphth-1-yl-methyl, Naphth-2-yl-methyl, Pyrid-2-yl-methyl, Pyrid-3-yl-methyl, Fur-2-yl-methyl, Fur-3-yl-methyl, Thien-2-yl-methyl, Thien-3-yl-methyl, Phenyl-C₁-C₄-alkyl, das gegebenenfalls durch einen oder mehrere gleiche oder verschiedene Reste der Reihe Halogen, Hydroxy, C₁-C₄-Alkyl, C₁-C₄-Alkoxy, Nitro, C₁-C₄-Alkylamino, C₁-C₄-Dialkylamino, C₃-C₆-Cycloalkoxyamino substituiert sein kann,
R⁷, R⁸ und R⁹ unabhängig voneinander für Wasserstoff, Methyl, Benzyl oder Cyclopropyl stehen,
B für Hydroxy oder tert.-Butyloxy steht,
sowie deren optische Isomere und Racemate.

6. Verwendung von offenkettigen Hexadepsipeptiden der Formel (I) gemäß Anspruch 1
in welcher
A für Wasserstoff, Benzyl, tert.-Butyloxycarbonyl (Boc) oder Benzyloxycarbonyl (Z) steht,
R¹, R³ und R⁵ unabhängig voneinander für Wasserstoff, Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, Cyclohexylmethyl, Phenylmethyl,
R², R⁴ und R⁶ unabhängig voneinander für Wasserstoff, Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Octyl, Isooctyl, sec.-Octyl, Vinyl, Allyl, Cyclohexylmethyl, Phenylmethyl, das gegebenenfalls durch einen oder mehrere gleiche oder verschiedene Reste der Reihe Halogen, C₁-C₄-Alkyl, C₁-C₄-Alkoxy, Nitro, C₁-C₄-Alkylamino, C₁-C₄-Dialkylamino, C₃-C₆-Cycloalkylamino C₃-c₆-Cycloalkoxyamino substituiert sein kann,
R⁷, R⁸ und R⁹ für Methyl stehen,
B für Hydroxy oder tert.-Butyloxy steht,
sowie deren optische Isomere und Racemate.

## Claims

1. Use of open-chain hexadepsipeptides of the general formula (I) 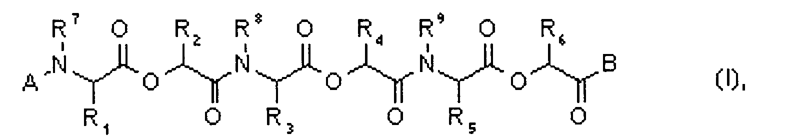 in which
A represents hydrogen, alkyl, aralkyl or an aryl radical, in particular a radical of the formula -CO-R¹⁰ in which
R¹⁰ represents straight-chain or branched alkyl, aralkyl, alkoxy or arylalkoxy having up to 6 carbon atoms in the alkyl moiety,
R¹, R³ and R⁵ independently of one another represent hydrogen, straight-chain or branched alkyl having up to 8 carbon atoms, hydroxyalkyl, alkanoyloxyalkyl, alkoxyalkyl, aryloxyalkyl, mercaptoalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, guanidinoalkyl which can optionally be substituted by one or two benzyloxycarbonyl radicals or by one, two, three or four alkyl radicals, or represent alkoxycarbonylaminoalkyl, 9-fluorenylmethoxycarbonyl(Fmoc)-aminoalkyl, alkenyl, cycloalkyl, cycloalkylalkyl, optionally substituted aryl, hetarylmethyl as well as arylalkyl, substituents which may be mentioned being halogen, hydroxyl, alkyl, alkoxy, nitro or a radical -NR¹¹R¹², where R¹¹ and R¹² independently of one another represent hydrogen or alkyl or R¹¹R¹² together with the adjacent N atom represent a carbocyclic 5-, 6- or 7-membered ring which can optionally also be interrupted by O, S and N and which is optionally substituted by C₁-C₄-alkyl,
R², R⁴ and R⁶ independently of one another represent hydrogen, straight-chain or branched alkyl having up to 8 carbon atoms, hydroxyalkyl, alkanoyloxyalkyl, alkoxyalkyl, aryloxyalkyl, alkylthioalkyl, alkylsulphinylalkyl, alkylsulphonylalkyl, carboxyalkyl, alkoxycarbonylalkyl, arylalkoxycarbonylalkyl, carbamoylalkyl, aminoalkyl, alkylaminoalkyl, dialkylaminoalkyl, alkoxycarbonylaminoalkyl, alkenyl, cycloalkyl, cycloalkylalkyl, hetarylmethyl and optionally substituted arylalkyl, substituents which may be mentioned being halogen, hydroxyl, alkyl, alkoxy, nitro or a radical -NR¹¹R¹², where R¹¹ and R¹² independently of one another represent hydrogen or alkyl or R¹¹R¹² together with the adjacent N atom represent a carbocyclic 5-, 6- or 7-membered ring which can optionally also be interrupted by O, S and N and which is optionally substituted by C₁-C₄-alkyl,
R⁷, R⁸ and R⁹ independently of one another represent hydrogen, C₁₋₈-alkyl, C₃₋₆-cycloalkyl or aralkyl, and
B represents hydroxyl or alkoxy having up to 4 carbon atoms,
and their optical isomers and racemates,
for the preparation of endoparasiticidal compositions for medicine and veterinary medicine.

2. Endoparasiticidal compositions, characterized in that they contain at least one open-chain hexadepsipeptide of the formula (I) according to Claim 1.

3. Process for the preparation of endoparasiticidal compositions, characterized in that open-chain hexadepsipeptides of the formula (I) according to Claim 1 are mixed with extenders and/or surfactants.

4. Use of open-chain hexadepsipeptides of the formula (I) according to Claim 1
in which
A represents hydrogen, C₁₋₄-alkyl, benzyl or represents a group of the formula -CO-R¹⁰
where
R¹⁰ represents straight-chain or branched C₁₋₄-alkyl, C₁₋₄-alkoxy or phenylalkoxy having up to 6 carbon atoms in the alkyl moiety,
R¹, R³ and R⁵ independently of one another represent hydrogen, straight-chain or branched C₁-C₈-alkyl, hydroxy-C₁-C₆-alkyl, C₁-C₄-alkanoyloxy-C₁-C₆-alkyl, C₁-C₄-alkoxy-C₁-C₆-alkyl, aryl-C₁-C₄-alkyloxy-C₁-C₆-alkyl, mercapto-C₁-C₆-alkyl, C₁-C₄-alkylthio-C₁-C₆-alkyl, C₁-C₄-alkylsulphinyl-C₁-C₆-alkyl, C₁-C₄-alkylsulphonyl-C₁-C₆-alkyl, carboxy-C₁-C₆alkyl, C₁-C₄-alkoxycarbonyl-C₁-C₆-alkyl, C₁-C₄-arylalkoxycarbonyl-C₁-C₆-alkyl, carbamoyl-C₁-C₆-alkyl, amino-C₁-C₆-alkyl, C₁-C₄-alkylamino-C₁-C₆-alkyl, C₁-C₄-dialkylamino-C₁-C₆-alkyl, guanido-C₁-C₆-alkyl, C₁-C₄-alkoxycarbonylamino-C₁-C₆-alkyl, 9-fluorenylmethoxycarbonyl(Fmoc)aminopropyl, 9-fluorenylmethoxycarbonyl(Fmoc)aminobutyl, C₂-C₈-alkenyl, C₃-C₇-cycloalkyl, C₃-C₇-cycloalkyl-C₁-C₄-alkyl, phenyl, benzo[b]thien-1-yl-methyl, benzo[b]thien-3-yl-methyl, naphth-1-yl-methyl, naphth-2-yl-methyl, pyrid-2-yl-methyl, pyrid-3-yl-methyl, fur-2-yl-methyl, fur-3-yl-methyl, thien-2-yl-methyl, thien-3-yl-methyl, indol-3-yl-methyl, N-methyl-indol-3-yl-methyl, imidazol-4-yl-methyl, N-methyl-imidazol-4-yl-methyl or Phenyl-C₁-C₄-alkyl which can optionally be substituted by radicals from the series consisting of halogen, hydroxyl, C₁-C₄-alkyl, C₁-C₄-alkoxy, methylenedioxy or ethylenedioxy, nitro or C₁-C₄-alkylamino, C₁-C₄-dialkylamino, C₃-C₆-cycloalkylamino, C₃-C₆-cycloalkoxyamino and C₃-C₆-cycloalkylthiooxyamino,
R², R⁴ and R⁶ independently of one another represent hydrogen, straight-chain or branched C₁-C₈-alkyl, hydroxyl-C₁-C₆-alkyl, C₁-C₄-alkanoyloxy-C₁-C₆-alkyl, C₁-C₄-alkoxy-C₁-C₆-alkyl, aryl-C₁-C₄-alkyloxy-C₁-C₆-alkyl, nercapto-C₁-C₆-alkyl, C₁-C₄-alkylthio-C₁-C₆-alkyl, C₁-C₄-alkylsulphinyl-C₁-C₆-alkyl, C₁-C₄-alkylsulphonyl-C₁-C₆-alkyl, carboxy-C₁-C₆-alkyl, C₁-C₄-alkoxycarbonyl-C₁-C₆-alkyl, C₁-C₄-arylalkoxycarbonyl-C₁-C₆-alkyl, carbamoyl-C₁-C₆-alkyl, amino-C₁-C₆-alkyl, C₁-C₄-alkylamino-C₁-C₆-alkyl, C₁-C₄-dialkylamino-C₁-C₆-alkyl, guanido-C₁-C₆-alkyl, C₁-C₄-alkoxycarbonylamino-C₁-C₆-alkyl, 9-fluorenylmethoxycarbonyl(Fmoc)aminopropyl, 9-fluorenylmethoxycarbonyl(Fmoc)aminobutyl, C₂-C₈-alkenyl, C₃-C₇-cycloalkyl, C₃-C₇-cycloalkyl-C₁-C₄-alkyl, phenyl, benzo[b]thien-1-yl-methyl, benzo[b]thien-3-yl-methyl, naphth-1-yl-methyl, naphth-2-yl-methyl, pyrid-2-yl-methyl, pyrid-3-yl-methyl, fur-2-yl-methyl, fur-3-yl-methyl, thien-2-yl-methyl, thien-3-yl-methyl, indol-3-yl-methyl, N-methylindol-3-yl-methyl, imidazol-4-yl-methyl or N-methylimidazol-4-yl-methyl, or represent phenyl-C₁-C₄-alkyl which can optionally be substituted by radicals from the series consisting of halogen, hydroxyl, C₁-C₄-alkyl, C₁-C₄-alkoxy, nitro or C₁-C₄-alkylamino, C₁-C₄-dialkylamino, C₃-C₆-cycloalkylamino, C₃-C₆-cycloalkoxyamino and C₃-C₆-cycloalkylthiooxyamino,
R⁷, R⁸ and R⁹ independently of one another represent hydrogen or straight-chain or branched C₁-C₄-alkyl, and
B represents hydroxyl or tert-butyloxy,
and their optical isomers and racemates.

5. Use of open-chain hexadepsipeptides of the formula (I) according to Claim 1
in which
A represents hydrogen or benzyl,
or represents a group of the formula -CO-R¹⁰
where
R¹⁰ represents straight-chain or branched alkoxy or arylalkoxy having up to 6 carbon atoms in the alkyl moiety,
R¹, R³ and R⁵ independently of one another represent hydrogen, straight-chain or branched C₁-C₈-alkyl, C₁-C₄-alkanoyloxy-C₁-C₆-alkyl, C₁-C₄-alkoxy-C₁-C₆-alkyl, aryl-C₁-C₄-alkyloxy-C₁-C₆-alkyl, C₁-C₄-alkoxycarbonylamino-C₁-C₆-alkyl, C₂-C₈-alkenyl, C₃-C₇-cycloalkyl, C₃-C₇-cycloalkyl-C₁-C₄-alkyl, phenyl, naphth-1-yl-methyl, pyrid-2-yl-methyl, pyrid-3-yl-methyl, thien-2-yl-methyl, thien-3-yl-methyl, indol-3-yl-methyl, N-methyl-indol-3-yl-methyl, imidazol-4-yl-methyl or N-methyl-imidazol-4-yl-methyl, or represent phenyl-C₁-C₄-alkyl which can optionally be substituted by one or more identical or different radicals from amongst those given in Claim 4,
R², R⁴ and R⁶ independently of one another represent hydrogen, straight-chain or branched C₁-C₈-alkyl, C₁-C₄-alkanoyloxy-C₁-C₆-alkyl, C₁-C₄-alkoxy-C₁-C₆-alkyl, aryl-C₁-C₄-alkyloxy-C₁-C₆-alkyl, C₁-C₄-alkoxy-carbonylamino-C₁-C₆-alkyl, C₂-C₈-alkenyl, C₃-C₇-cycloalkyl, C₃-C₇-cycloalkyl-C₁-C₄-alkyl, phenyl, benzo[b]thien-2-yl-methyl, naphth-1-yl-methy], naphth-2-yl-methyl, pyrid-2-yl-methyl, pyrid-3-yl-methyl, fur-2-yl-methyl, fur-3-yl-methyl, thien-2-yl-methyl or thien-3-yl-methyl, or represent phenyl-C₁-C₄-alkyl which can optionally be substituted by one or more identical or different radicals from the series consisting of halogen, hydroxyl, C₁-C₄-alkyl, C₁-C₄-alkoxy, nitro, C₁-C₄-alkylamino, C₁-C₄-dialkylamino and C₃-C₆-cycloalkoxyamino,
R⁷, R⁸ and R⁹ independently of one another represent hydrogen, methyl, benzyl or cyclopropyl, and
B represents hydroxyl or tert-butyloxy,
and their optical isomers and racemates.

6. Use of open-chain hexadepsipeptides of the formula (I) according to Claim 1
in which
A represents halogen, benzyl, tert-butyloxycarbonyl (Boc) or benzyloxycarbonyl (Z),
R¹, R³ and R⁵ independently of one another represent hydrogen, methyl, ethyl, propyl, isopropyl, butyl, isobutyl, sec-butyl, pentyl, isopentyl, sec-pentyl, hexyl, isohexyl, sec-hexyl, heptyl, isoheptyl, sec-heptyl, octyl, isooctyl, sec-octyl, cyclohexylmethyl or phenylmethyl,
R², R⁴ and R⁶ independently of one another represent hydrogen, methyl, ethyl, propyl, isopropyl, butyl, isobutyl, sec-butyl, pentyl, isopentyl, sec-pentyl, hexyl, isohexyl, sec-hexyl, heptyl, isoheptyl, sec-heptyl, octyl, isooctyl, sec-octyl, vinyl, allyl or cyclohexylmethyl, or represent phenylmethyl which can optionally be substituted by one or more identical or different radicals from the series consisting of halogen, C₁-C₄-alkyl, C₁-C₄-alkoxy, nitro, C₁-C₄-alkylamino, C₁-C₄-dialkylamino, C₃-C₆-cycloalkylamino and C₃-C₆-cycloalkoxyamino,
R⁷, R⁸ and R⁹ represent methyl, and
B represents hydroxyl or tert-butyloxy,
and their optical isomers and racemates.

## Revendications

1. Utilisation d'hexadepsipeptides à chaîne ouverte de formule générale (I) : 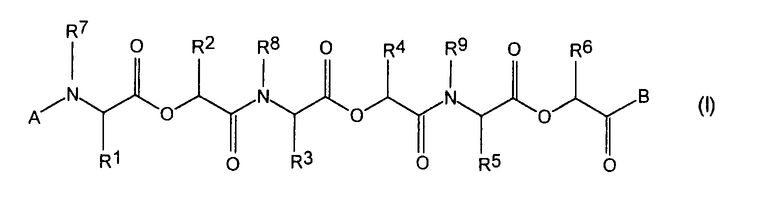 dans laquelle :
A représente un reste hydrogène, alcoyle, aralcoyle ou aryle, en particulier un radical de formule -CO-R¹⁰, où
R¹⁰ représente un alcoyle, aralcoyle linéaire ou ramifié, alcoxy ou aralcoxy ayant jusqu'à 6 atomes de carbone dans la partie alcoyle,
R¹, R³ et R⁵ représentent indépendamment l'un de l'autre, hydrogène, alcoyle linéaire ou ramifié ayant jusqu'à 8 atomes de carbone, hydroxyalcoyle, alcanoyloxyalcoyle, alcoxyalcoyle, aryloxyalcoyle, mercaptoalcoyle, alcoylthioalcoyle, alcoylsulfinylalcoyle, alcoylsulfonylalcoyle, carboxyalcoyle, alcoxycarbonylalcoyle, arylalcoxycarbonylalcoyle, carbamoylalcoyle, aminoalcoyle, alcoylaminoalcoyle, dialcoylaminoalcoyle, guanidinoalcoyle, qui peut être facultativement substitué par un ou deux restes benzyloxycarbonyle ou par un, deux, trois ou quatre restes alcoyle, alcoxycarbonylaminoalcoyle, 9-fluorénylméthoxycarbonyl(Fmoc)aminoalcoyle, alcényle, cycloalcoyle, cycloalcoylalcoyle, aryle facultativement substitué, hétarylméthyle ainsi que arylalcoyle, où comme substituants, on citera halogène, hydroxy, alcoyle, alcoxy, nitro ou un radical -NR¹¹R¹², où R¹¹ et R¹² représentent indépendamment l'un de l'autre, hydrogène ou alcoyle, ou R¹¹R¹², ensemble avec l'atome N voisin, représentent un carbocycle à 5, 6 ou 7 membres, lequel peut également être facultativement interrompu par O, S et N et peut facultativement être substitué par alcoyle en C₁-C₄ ;
R², R⁴ et R⁶ représentent indépendamment l'un de l'autre, hydrogène, alcoyle linéaire ou ramifié ayant jusqu'à 8 atomes de carbone, hydroxyalcoyle, alcanoyloxyalcoyle, alcoxyalcoyle, aryloxyalcoyle, alcoylthioalcoyle, alcoylsulfinylalcoyle, alcoylsulfonylalcoyle, carboxyalcoyle, alcoxycarbonylalcoyle, arylalcoxycarbonylalcoyle, carbamoylalcoyle, aminoalcoyle, alcoylaminoalcoyle, dialcoylaminoalcoyle, alcoxycarbonylaminoalcoyle, alcényle, cycloalcoyle, cycloalcoylalcoyle, hétarylméthyle ainsi que arylalcoyle facultativement substitué, où comme substituants, on citera halogène, hydroxy, alcoyle, alcoxy, nitro ou un radical -NR¹¹R¹², où R¹¹ et R¹² représentent indépendamment l'un de l'autre, hydrogène ou alcoyle, ou R¹¹R¹², ensemble avec l'atome N voisin, représentent un carbocycle à 5, 6 ou 7 membres, lequel peut également être facultativement interrompu par O, S et N et peut facultativement être substitué par alcoyle en C₁-C₄ ;
R⁷, R⁸ et R⁹ représentent indépendamment l'un de l'autre, hydrogène, alcoyle en C₁₋₈, cycloalcoyle en C₃₋₆ ou aralcoyle ;
B représente hydroxy ou alcoxy ayant jusqu'à 4 atomes de carbone,
ainsi que leurs isomères optiques et racémates,
pour préparer des agents endoparasiticides à utiliser en médecine humaine et vétérinaire.

2. Agent endoparasiticide, caractérisé en ce qu'il contient au moins un hexadepsipeptide à chaîne ouverte, de formule (I) suivant la revendication 1.

3. Procédé de préparation d'agents endoparasiticides, caractérisé en ce que l'on mélange un hexadepsipeptide à chaîne ouverte, de formule (I) suivant la revendication 1, avec des diluants et/ou agents tensioactifs.

4. Utilisation d'hexadepsipetides à chaîne ouverte de formule (I), suivant la revendication 1, dans laquelle :
A représente hydrogène, alcoyle en C₁₋₄, benzyle ou un radical de formule -CO-R^{10,} où
R¹⁰ représente alcoyle en C₁₋₄ linéaire ou ramifié, alcoxy en C₁₋₄ ou phénylalcoxy ayant jusqu'à 6 atomes de carbone dans la partie alcoyle ;
R¹, R³ et R⁵ représentent indépendamment l'un de l'autre, hydrogène, alcoyle en C₁₋₈ linéaire ou ramifié, hydroxyalcoyle en C₁₋₆, (alcanoyl en C₁₋₄)oxyalcoyle en C₁₋₆, (alcoxy en C₁₋₄)alcoyle en C₁₋₆, aryl(alcoyloxy en C₁₋₄)alcoyle en C₁₋₆, mercaptoalcoyle en C₁₋₆, (alcoylthio en C₁₋₄)alcoyle en C₁₋₆, (alcoylsulfinyl en C₁₋₄)alcoyle en C₁₋₆, (alcoylsulfonyl en C₁₋₄)alcoyle en C₁₋₆, carboxyalcoyle en C₁₋₆, (alcoxy en C₁₋₄) carbonyl(alcoyle en C₁₋₆), (arylalcoxycarbonyl en C₁₋₄)alcoyle en C₁₋₆, carbamoylalcoyle en C₁₋₆, aminoalcoyle en C₁₋₆, (alcoylamino en C₁₋₄)alcoyle en C₁₋₆, di(alcoyl en C₁₋₄)aminoalcoyle en C₁₋₆, guanidinoalcoyle en C₁₋₆, (alcoxy en C₁₋₄)carbonylaminoalcoyle en C₁₋₆, 9-fluorénylméthoxycarbonyl(Fmoc)aminopropyle, 9-fluorénylméthoxycarbonyl(Fmoc)aminobutyle, alcényle en C₂₋₈, cycloalcoyle en C₃₋₇, (cycloalcoyl en C₃₋₇)alcoyle en C₁₋₄, phényle, benzo[b]thièn-1-ylméthyle, benzo[b]thièn-3-ylméthyle, napht-1-ylméthyle, napht-2-ylméthyle, pyrid-2-ylméthyle, pyrid-3-ylméthyle, fur-2-ylméthyle, fur-3-ylméthyle, thièn-2-ylméthyle, thièn-3-ylméthyle, indol-3-ylméthyle, N-méthylindol-3-ylméthyle, imidazol-4-ylméthyle, N-méthylimidazol-4-ylméthyle, phénylalcoyle en C₁₋₄, qui peut être facultativement substitué par des restes de la série des halogènes, hydroxy, alcoyle en C₁₋₄, alcoxy en C₁₋₄, méthylènedioxy ou éthylènedioxy, nitro ou alcoylamino en C₁₋₄, di(alcoyle en C₁₋₄)amino, cycloalcoylamino en C₃₋₆, cycloalcoxyamino en C₃₋₆, cycloalcoylthioxyamino en C₃₋₆ ;
R², R⁴ et R⁶ représentent indépendamment l'un de l'autre, hydrogène, alcoyle en C₁₋₈ linéaire ou ramifié, hydroxyalcoyle en C₁₋₆, (alcanoyl en C₁₋₄)oxyalcoyle en C₁₋₆, (alcoxy en C₁₋₄)alcoyle en C₁₋₆, aryl(alcoyloxy en C₁₋₄)alcoyle en C₁₋₆, mercaptoalcoyle en C₁₋₆, (alcoylthio en C₁₋₄ alcoyle en C₁₋₆, (alcoylsulfinyl en C₁₋₄) alcoyle en C₁₋₆, (alcoylsulfonyl en C₁₋₄)alcoyle en C₁₋₆, carboxyalcoyle en C₁₋₆, (alcoxy en C₁₋₄) carbonyl(alcoyle en C₁₋₆), (arylalcoxycarbonyl en C₁₋₄)alcoyle en C₁₋₆, carbamoylalcoyle en C₁₋₆, aminoalcoyle en C₁₋₆, (alcoylamino en C₁₋₄)alcoyle en C₁₋₆, di(alcoyl en C₁₋₄)aminoalcoyle en C₁₋₆, guanidinoalcoyle en C₁₋₆, (alcoxy en C₁₋₄)carbonylaminoalcoyle en C₁₋₆, 9-fluorénylméthoxycarbonyl(Fmoc)aminopropyle, 9-fluorénylméthoxycarbonyl(Fmoc)aminobutyle, alcényle en C₂₋₈, cycloalcoyle en C₃₋₇, (cycloalcoyl en C₃₋₇)alcoyle en C₁₋₄, phényle, benzo[b]thièn-1-ylméthyle, benzo[b]thièn-3-ylméthyle, napht-1-ylméthyle, napht-2-ylméthyle, pyrid-2-ylméthyle, pyrid-3-ylméthyle, fur-2-ylméthyle, fur-3-ylméthyle, thièn-2-ylméthyle, thièn-3-ylméthyle, indol-3-ylméthyle, N-méthylindol-3-ylméthyle, imidazol-4-ylméthyle, N-méthylimidazol-4-ylméthyle, phénylalcoyle en C₁₋₄, qui peut être facultativement substitué par des restes de la série des halogènes, hydroxy, alcoyle en C₁₋₄, alcoxy en C₁₋₄, nitro ou alcoylamino en C₁₋₄, di(alcoyle en C₁₋₄)amino, cycloalcoylamino en C₃₋₆, cycloalcoxyamino en C₃₋₆, cycloalcoylthioxyamino en C₃₋₆ ;
R⁷, R⁸ et R⁹ représentent indépendamment l'un de l'autre, hydrogène, alcoyle en C₁₋₄ linéaire ou ramifié ;
B représente hydroxy ou t-butyloxy ;
ainsi que leurs isomères optiques et racémates.

5. Utilisation d'hexadepsipetides à chaîne ouverte de formule (I), suivant la revendication 1, dans laquelle :
A représente hydrogène ou benzyle ou un radical de formule -CO-R^{10,} où
R¹⁰ représente alcoxy linéaire ou ramifié, ou arylalcoxy ayant jusqu'à 6 atomes de carbone dans la partie alcoyle ;
R¹, R³ et R⁵ représentent indépendamment l'un de l'autre, hydrogène, alcoyle en C₁₋₈ linéaire ou ramifié, (alcanoyl en C₁-₄)oxyalcoyle en C₁-₆, (alcoxy en C₁₋₄)alcoyle en C₁₋₆, aryl(alcoyloxy en C₁₋₄)alcoyle en C₁₋₆, (alcoxy en C₁₋₄)carbonylamino(alcoyle en C₁₋₆), alcényle en C₂₋₈, cycloalcoyle en C₃₋₇, (cycloalcoyl en C₃-₇)alcoyle en C₁₋₄, phényle, napht-1-ylméthyle, pyrid-2-ylméthyle, pyrid-3-ylméthyle, thièn-2-ylméthyle, thièn-3-ylméthyle, indol-3-ylméthyle, N-méthylindol-3-ylméthyle, imidazol-4-ylméthyle, N-méthylimidazol-4-ylméthyle, phénylalcoyle en C₁₋₄, qui peut être facultativement substitué par un ou plusieurs restes identiques ou différents donnés à la revendication 4 ;
R², R⁴ et R⁶ représentent indépendamment l'un de l'autre, hydrogène, alcoyle en C₁₋₈ linéaire ou ramifié, (alcanoyl en C₁₋₄)oxyalcoyle en C₁₋₆, (alcoxy en C₁₋₄)alcoyle en C₁₋₆, aryl(alcoyloxy en C₁₋₄ alcoyle en C₁₋₆, (alcoxy en C₁₋₄)carbonylamino(alcoyle en C₁₋₆), alcényle en C₂₋₈, cycloalcoyle en C₃₋₇, (cycloalcoyl en C₃₋₇)alcoyle en C₁₋₄, phényle, benzo[b]thièn-2-ylméthyle, napht-1-ylméthyle, napht-2-ylméthyle, pyrid-2-ylméthyle, pyrid-3-ylméthyle, fur-2-ylméthyle, fur-3-ylméthyle, thièn-2-ylméthyle, thièn-3-ylméthyle, phénylalcoyle en C₁₋₄, qui peut être facultativement substitué par un ou plusieurs restes indentiques ou différents, de la série des halogènes, hydroxy, alcoyle en C₁₋₄, alcoxy en C₁₋₄, nitro ou alcoylamino en C₁₋₄, di(alcoyle en C₁₋₄)amino, cycloalcoxyamino en C₃₋₆, ;
R⁷, R⁸ et R⁹ représentent indépendamment l'un de l'autre, hydrogène, méthyle, benzyle ou cyclopropyle ;
B représente hydroxy ou t-butyloxy ;
ainsi que leurs isomères optiques et racémates.

6. Utilisation d'hexadepsipetides à chaîne ouverte de formule (I), suivant la revendication 1, dans laquelle :
A représente hydrogène, benzyle, t-butyloxycarbonyl (Boc) ou benzyloxycarbonyl (Z) ;
R¹, R³ et R⁵ représentent indépendamment l'un de l'autre hydrogène, méthyle, éthyle, propyle, isopropyle, butyle, isobutyle, s-butyle, pentyle, isopentyle, s-pentyle, hexyle, isohexyle, s-hexyle, heptyle, isoheptyle, s-heptyle, octyle, isooctyle, s-octyle, cyclohexylméthyle, phénylméthyle ;
R², R⁴ et R⁶ représentent indépendamment l'un de l'autre, hydrogène, méthyle, éthyle, propyle, isopropyle, butyle, isobutyle, s-butyle, pentyle, isopentyle, s-pentyle, hexyle, isohexyle, s-hexyle, heptyle, isoheptyle, s-heptyle, octyle, isooctyle, s-octyle, vinyle, allyle, cyclohexylméthyle, phénylméthyle qui peut être facultativement substitué par un ou plusieurs restes identiques ou différents, de la série des halogènes, alcoyle en C₁₋₄, alcoxy en C₁₋₄, nitro, alcoylamino en C₁₋₄, di(alcoyle en C₁₋₄)amino, cycloalcoxyamino en C₃₋₆, ;
R⁷, R⁸ et R⁹ représentent méthyle ;
B représente hydroxy ou t-butyloxy ;
ainsi que leurs isomères optiques et racémates.
